(19) Europäisches Patentamt
European Patent Office
Office européen des brevets

(11) EP 3 772 543 B1

(12) **EUROPEAN PATENT SPECIFICATION**

(45) Date of publication and mention
of the grant of the patent:
**27.09.2023 Bulletin 2023/39**

(21) Application number: **19190989.4**

(22) Date of filing: **09.08.2019**

(51) International Patent Classification (IPC):
***C12Q 1/6886*** *(2018.01)*

(52) Cooperative Patent Classification (CPC):
(C-Sets available)
**C12Q 1/6886; C12N 15/1017; C12Q 1/6806;
G01N 33/5076; G01N 33/57415; C12Q 2600/178**

(Cont.)

(54) **METHOD FOR DIAGNOSING BREAST CANCER**

VERFAHREN ZUR DIAGNOSE VON BRUSTKREBS

PROCÉDÉ DE DIAGNOSTIC DU CANCER DU SEIN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**10.02.2021 Bulletin 2021/06**

(73) Proprietor: **Albert-Ludwigs-Universität Freiburg
79098 Freiburg (DE)**

(72) Inventors:
• **JÄGER, Markus
79238 Ehrenkirchen (DE)**
• **HIRSCHFELD, Marc
37445 Walkenried (DE)**
• **ERBES, Thalia
79194 Gundelfingen (DE)**
• **WEIß, Daniela
79312 Emmendingen (DE)**

(74) Representative: **Lederer & Keller Patentanwälte
Partnerschaft mbB
Unsöldstraße 2
80538 München (DE)**

(56) References cited:
EP-A1- 3 070 178     WO-A1-2016/144265
US-A1- 2010 286 044

• THALIA ERBES ET AL: "Feasibility of urinary microRNA detection in breast cancer patients and its potential as an innovative non-invasive biomarker", BMC CANCER, BIOMED CENTRAL, LONDON, GB, vol. 15, no. 1, 28 March 2015 (2015-03-28) , page 193, XP021220281, ISSN: 1471-2407, DOI: 10.1186/S12885-015-1190-4
• ZHANG LEI ET AL: "A circulating miRNA signature as a diagnostic biomarker for non-invasive early detection of breast cancer", BREAST CANCER RESEARCH AND TREATMENT, SPRINGER , NY, US, vol. 154, no. 2, 17 October 2015 (2015-10-17), pages 423-434, XP035902260, ISSN: 0167-6806, DOI: 10.1007/S10549-015-3591-0 [retrieved on 2015-10-17]
• RUTH RODRIGUEZ-BARRUECO ET AL: "miR-424(322)/503 is a breast cancer tumor suppressor whose loss promotes resistance to chemotherapy", GENES & DEVELOPMENT, vol. 31, 1 January 2017 (2017-01-01), pages 553-566, XP055663195, DOI: 10.1101/gad.292318

Remarks:
The complete document including Reference Table(s) and the Sequence Listing(s) can be downloaded from the EPO website

(52) Cooperative Patent Classification (CPC): (Cont.)

C-Sets
**C12Q 1/6806, C12Q 2527/156**

**Description**

BACKGROUND OF THE INVENTION

The distinct impact of breast cancer (BC) on female health and associated life expectancy is reflected by a continuously increasing number of global incidence rates during the course of the past decades. Ascertainment of cancer registry data of 102 countries in the period of 1990 - 2016 as part of the Global Burden of Disease (GBD) study [1] identified breast cancer (BC) as the most frequent malignant disease in women with 1.7 million worldwide cases in 2016 [2]. Malignancies originating from breast tissue account for 25% of all female cancers [3]. Since 1990 the BC incidence rates more than doubled in 60/102 countries, affecting both, developed and developing regions [2]. Early detection of BC is the decisive factor for the cure rate [4-6] although BC-associated mortality rates could be reduced in general due to improved screening and treatment options, heterogeneous quality pattern in different countries sustain a crucial weak point in BC management [2, 7]. Routine BC detection methods including palpation, mammography and ultrasound are characterized by various limitations, e.g. moderate sensitivity and specificity rates especially at denser mammary tissue, decreased patient compliance [8, 9]. Thus, there is a huge demand for methodical innovation for improved (early) diagnosis of breast cancer.

Liquid biopsy-based disease biomarkers offer a range of promising prospects as important prognostic, diagnostic and theranostic tools [10]. Various biomarker types are available in body liquids via minimal- or non-invasive sampling procedures [10]. Among others, circulating microRNA (miRNA / miR) molecules qualify as robust and reliable matrices in regard to detectability and specificity in disease diagnosis and monitoring [10, 11]. Recently, the feasibility of urinary miRs in breast cancer detection could be demonstrated [12]. Similarly, the applicability of urinary miRs with diagnostic features was proven also in other tumor types [10, 13-15]. Subsequent to the isolation of miRs from exosomes in urine specimen, expression analysis of these small nucleic acids provides distinct characteristic pattern to distinguish cancer patients from healthy controls [12, 14, 16].

Dysregulated expression of let-7-family members of miRNAs could be linked to breast carcinogenesis [17-22]. Distinct let-7 miRNAs bear the potential to be employed as prospective molecular markers in BC diagnostics and as therapeutic targets [22-25].

The functional involvement and putative diagnostic implications of miR-17 in BC could be demonstrated in several studies [26-29] with one pointing out the predictive potential in BC recurrence management [30]. Aberrant expression levels of miR-125 relate to BC tumorigenesis, within which it displays a functional correlation with the HER2-overexpressing BC subtype influencing the corresponding metastatic risk and prognosis [31-34]. Malignant breast tissue transformation was found accompanied by miR-222 activities regulating decisive signaling pathways in tumor progression [35-37]. Most interestingly, miR-222 potentially offers predictive power in hormone receptor-positive BC [38]. Exosome-mediated intercellular transfer of miR-222 was found associated with BC metastasis [39]. With focus on BC, the biomarker potential of miR-107 could be demonstrated in various functional studies, which also revealed its impact on BRCA1 activity and BC recurrence prediction in triple-negative BC (TNBC) [40-45]. Among others, the levels of circulating miR-107 were found correlated with lymph node metastasis and receptor status in BC patients [46]. Wnt/$\beta$-catenin signaling pathway in BC was found regulated by miR-194, thereby affecting cancer cell proliferation, migration and invasion [47]. Circulating miR-194 molecules could be linked to BC recurrence risk [48], but also exhibited a functional interrelation with the anti-HER2 agent trastuzumab [49].

The functional background of miR-423 and the crucial impact of altered SNPs (single nucleotide polymorphisms) in the miR-423 gene on BC biology became apparent in recent investigations [50, 51]. Another functional aspect is provided by the combined expression levels of miR-423 and miR-4417, which could differentiate 70.1 % of hereditary and non-hereditary BCs [52].

Aside from cancer-relevant regulatory impact in basal-like BC [53], tumor suppressor activities influencing BC chemotherapy resistance could be described for miR-424 [54]. A serum-based BC biomarker study could identify a 3-miR signature comprising the expression levels of miR-424, miR-29c, and miR-199a with the highest diagnostic accuracy for distinguishing breast cancer patients from healthy controls [55]. Oncogenic effects could be linked to miR-660 expression, with triggering functions in BC proliferation, migration and anti-apoptotic activities [56]. A BC tissue-derived next generation sequencing analysis extracted miR-660 as one promising biomarker candidate with significant prognostic features in overall survival (OS) and recurrence-free survival (RFS) in BC patients [57]. In this study, expression levels of a set of thirteen distinct miRNA types was analyzed in the urine of untreated BC patients compared to healthy controls in order to extract a non-invasive biomarker tool applicable for BC diagnosis. EP 3070178 A1 describes studies on urine samples of breast cancer patients. It was found that the urinary miRNAs miR-21, miR-125b, miR-375 and miR-451 displayed significantly decreased expression levels in breast cancer patients compared to healthy controls. In addition, the urinary miRNA miR-155 showed an increased expression level in breast cancer patients. EP 3070178 A1 suggests that in particular determination of the expression level of the four miRNAs miR-21, miR-125b, miR-155 and miR-451 in urine samples allows for a discrimination between healthy individuals and BC patients.

There is an ongoing need for improved methods of diagnosing breast cancer.

SUMMARY OF THE INVENTION

The inventors investigated urine samples of breast cancer patients and surprisingly found that certain miRNA types and combinations thereof are useful in diagnosing breast cancer. In particular, a panel of miRNA types, comprising at least **miR-424, miR-660,** and **let7-i,** was found to be a highly specific combinatory biomarker tool in discrimination of breast cancer patients vs. healthy controls based on urine specimen. The inventors further developed a highly efficient method for isolating exosomes from urine and other body fluids, which is particularly useful in the method for diagnosing BC described herein.

BRIEF DESCRIPTION OF THE DRAWINGS

**Figure 1**: NRQ values of miR expression in urine specimen of BC patients.
**Figure 2:** NRQ values of miR expression in urine specimen of healthy controls.
Figure 3: Coefficient paths for the stepwise boosting procedure. The x-axis shows the boosting steps (here 1 to 1500), the y-axis shows the coefficient estimates for each selected variable. After 1500 steps, seven variables had been selected.
**Figure 4:** Workflow scheme on microvesicle isolation from body fluids.
**Figure 5:** Equipment assembly of filtration method.
**Figure 6:** Western Blot showing the result of Example 2.

DETAILED DESCRIPTION

The present invention encompasses methods of diagnosing whether a subject has, or is at risk for developing, breast cancer, comprising determining the level of miR gene products in a urine sample from the subject and comparing the level of the miR gene products in the urine sample to the level of the respective miR gene products in a control sample.

In particular, the invention relates to a method of diagnosing whether a subject has cancer (e.g. breast cancer), comprising determining the level of miR gene products in a urine sample from the subject, wherein an increase or a decrease in the level of the miR gene products in the urine sample, relative to the level of the respective miR gene products in a control sample, is indicative of the subject having cancer (e.g. breast cancer), wherein the miR gene products are miR-424, miR-660, let7-i.

As used herein interchangeably, a "miR gene product," "microRNA," "miR," or "miRNA" refers to the unprocessed (e.g., precursor) or processed (e.g., mature) RNA transcript from a miR gene. As the miR gene products are not translated into protein, the term "miR gene products" does not include proteins. The unprocessed miR gene transcript is also called a "miR precursor" or "miR prec" and typically comprises an RNA transcript of about 70-100 nucleotides in length. The miR precursor can be processed by digestion with an RNAse (for example, Dicer, Argonaut, or RNAse III (e.g., E. coli RNAse III)) into an active 19-25 nucleotide RNA molecule. This active 19-25 nucleotide RNA molecule is also called the "processed" miR gene transcript or "mature" miRNA. The active 19-25 nucleotide RNA molecule can be obtained from the miR precursor through natural processing routes (e.g., using intact cells or cell lysates) or by synthetic processing routes (e.g., using isolated processing enzymes, such as isolated Dicer, Argonaut, or RNAse III). It is understood that the active 19-25 nucleotide RNA molecule can also be produced directly by biological or chemical synthesis, without having been processed from the miR precursor. When a microRNA is referred to herein by name, the name corresponds to both the precursor and mature forms, unless otherwise indicated. Table 1 depicts the nucleotide sequences of preferred mature human microRNAs used in the present invention.

Table 1

| Name of microRNA | Mature sequence | SEQ ID NO: of mature sequence |
|---|---|---|
| miR-423 | UGAGGGGCAGAGAGCGAGACUUU | 10 |
| miR-424 | CAGCAGCAAUUCAUGUUUUGAA | 11 |
| miR-660 | UACCCAUUGCAUAUCGGAGUUG | 12 |
| let7-i | UGAGGUAGUAGUUUGUGCUGUU | 17 |
| miR-125b | UCCCUGAGACCCUAACUUGUGA | 7 |

(continued)

| Name of microRNA | Mature sequence | SEQ ID NO: of mature sequence |
|---|---|---|
| let7-d | AGAGGUAGUAGGUUGCAUAGUU | 14 |
| miR-17 | CAAAGUGCUUACAGUGCAGGUAG | 4 |
| let7-f | UGAGGUAGUAGAUUGUAUAGUU | 16 |
| miR194 | UGUAACAGCAACUCCAUGUGGA | 8 |
| miR222 | AGCUACAUCUGGCUACUGGGU | 9 |

As used herein, a "subject" can be any mammal that has, or is suspected of having, breast cancer. In a preferred embodiment, the subject is a human who has, or is suspected of having, breast cancer. Most preferably, the subject is a female human, e.g. a woman at an age of 30 to 70 years, 35 to 69 years, 40 to 67 years, or 45 to 65 years.

The term "urine sample" refers to a sample which comprises or consists of urine, or which is derived from urine. For determining the level of miR gene products in the urine sample, native urine is typically processed prior to analysis. The processing may include (but is not limited to) RNA isolation and/or purification, centrifugation, nucleic acid precipitation, dissolution of precipitated nucleic acid, concentration, dilution, filtration and combinations thereof. Such processed samples are encompassed by the term "urine sample". In one embodiment the urine sample to be analysed is obtainable or obtained by enriching or isolating extracellular vesicles from urine. In another embodiment the urine sample is obtainable or obtained by enriching or isolating exosomes from urine. In preferred embodiments the urine sample to be analysed is obtainable or obtained by a method for isolating exosomes described hereinbelow.

As used herein, the term "extracellular vesicles" (EVs) refers to membrane-contained vesicles released by cells. EVs can be broadly classified into 3 main classes: (a) Microvesicles (or microparticles or ectosomes) that are produced by outward budding and fission of the plasma membrane; (b) Exosomes that are formed within the endosomal network and released upon fusion of multi-vesicular bodies with the plasma membrane; and (c) Apoptotic bodies which are released as blebs of cells undergoing apoptosis.

The level of miR gene products is determined in a urine sample obtained from the subject. A corresponding control sample, which is also a urine sample, can be obtained from a healthy human individual or population of healthy individuals. The healthy "control" individual preferably has the same gender as the subject, and optionally a similar age (i.e. +/- 10 years the age of the subject). Most preferably, the "control" individual is a healthy woman at an age of 30 to 70 years, 35 to 69 years, 40 to 67 years, or 45 to 65 years. The control urine sample is then processed along with the urine sample from the subject, so that the levels of miR gene product in the subject's urine sample can be compared to the respective miR gene product levels in the control sample. A reference miR expression standard for the urine sample can also be used as a control.

An alteration (e.g., an increase or decrease) in the level of a miR gene product in the sample obtained from the subject, relative to the level of the respective miR gene product in a control sample, as described below, is indicative of the presence of breast cancer in the subject. In one embodiment, the level of the at least one miR gene product in the test sample is greater than the level of the respective miR gene product in the control sample (i.e., expression of the miR gene product is "up-regulated"). As used herein, expression of a miR gene product is "up-regulated" when the amount of miR gene product in a urine sample from a subject is greater than the amount of the same gene product in a control sample.

In another embodiment, the level of the at least one miR gene product in the urine sample is less than the level of the respective miR gene product in the control sample (i.e., expression of the miR gene product is "down-regulated"). As used herein, expression of a miR gene is "down-regulated" when the amount of miR gene product produced from that gene in a urine sample from a subject is less than the amount produced from the same gene in a control sample.

The relative miR gene expression in the control and normal samples can be determined with respect to one or more RNA expression standards. The standards can comprise, for example, the urinary miR gene expression level in healthy subject, or the average level of urinary miR gene expression previously obtained for a population of healthy human controls. "Healthy" means that the subject or human controls do not have breast cancer or another type of cancer.

The method comprises determining the level of a miR-424 gene product, a miR-660 gene product and a let7-i gene product, wherein a decrease in the levels of the let7-i and miR-660 gene products in the urine sample, relative to the respective levels of the respective miR gene products in a control sample, and an increase in the level of the miR-424 gene product in the urine sample, relative to the level of the miR-424 gene product in the control sample, is indicative of the subject having breast cancer.

*Determining the level of miR gene products*

The level of a miR gene product in a urine sample can be measured using any technique that is suitable for detecting RNA expression levels in a urine sample. Nucleic acids can used be as probes or primers for embodiments involving nucleic acid hybridization. As such, they may be used to assess miRNA expression. The nucleotide sequences of the invention may be used for their ability to selectively form duplex molecules with complementary stretches of DNAs and/or RNAs or to provide primers for amplification of DNA or RNA from samples.

Preferably the processing efficiency in regard to RNA extraction, complementary DNA synthesis (reverse transcription) and PCR amplification is monitored by addition of "spike-in" control RNA specimen of defined concentration to the RNA lysis buffer as recommended by Marabita [4]. The detailed procedure is decribed herein in section 1.6 of Example 1.

In another preferred embodiment the amount of each miR gene product is normalized by converting it into normalized relative quantities (NRQ) for the respective miR gene product. In that embodiment the NRQ is used as the level of miR gene product.

Reverse transcription (RT) of RNA to cDNA followed by quantitative PCR (RT-PCR) can be used to determine the relative concentrations of specific miRNA species. By determining that the concentration of a specific mRNA species varies, it is shown that the gene encoding the specific mRNA species is differentially expressed. Another method for amplification is ligase chain reaction ("LCR"). U.S. Pat. No. 4,883,750 describes a method similar to LCR for binding probe pairs to a target sequence. A method based on PCR™ and oligonucleotide ligase assay (OLA), disclosed in U.S. Pat. No. 5,912,148, may also be used. Alternative methods for amplification of target nucleic acid sequences that may be used in the practice of the present invention are disclosed in U.S. Pat. Nos. 5,843,650, 5,846,709, 5,846,783, 5,849,546, 5,849,497, 5,849,547, 5,858,652, 5,866,366, 5,916,776, 5,922,574, 5,928,905, 5,928,906, 5,932,451, 5,935,825, 5,939,291 and 5,942,391, GB Application No. 2 202 328, and in PCT Application No. PCT/US89/01025, each of which is incorporated herein by reference in its entirety. Alternatively, digital PCT or digital droplet PCR or next generation sequencing can be used to determine the level of miR gene product in the urine sample. Any other suitable method for determining the amount of micro RNA can be used.

Alternatively, an oligolibrary, in microchip format (i.e., a microarray), may be constructed containing a set of oligonucleotide probes that are specific for a set of miR genes. Using such a microarray, the expression level of multiple microRNAs in a urine sample can be determined by reverse transcribing the RNAs to generate a set of target oligodeoxynucleotides, and hybridizing them to probe the oligonucleotides on the microarray to generate a hybridization, or expression, profile. The hybridization profile of the test sample can then be compared to that of a control sample to determine which microRNAs have an altered expression level in breast cancer cells. As used herein, "probe oligonucleotide" or "probe oligodeoxynucleotide" refers to an oligonucleotide that is capable of hybridizing to a target oligonucleotide. "Target oligonucleotide" or "target oligodeoxynucleotide" refers to a molecule to be detected (e.g., via hybridization). By "miR-specific probe oligonucleotide" or "probe oligonucleotide specific for a miR" is meant a probe oligonucleotide that has a sequence selected to hybridize to a specific miR gene product, or to a reverse transcript of the specific miR gene product.

The method of the invention may further comprise the step of comparing the level of the miR gene product in the urine sample to a control level of the miR gene product (e.g. the level in a control sample), and diagnosing whether the subject has breast cancer, wherein a decrease or increase of miR gene product in the urine sample, relative to the control level of miR gene product is indicative of the subject having breast cancer.

Breast cancer is a cancer that starts in the breast, usually in the inner lining of the milk ducts or lobules. There are different types of breast cancer, with different stages (spread), aggressiveness, and genetic makeup. Breast cancer subtypes may be categorized on an immunohistochemical basis. The breast cancer to be diagnosed, detected, monitored or screened in accordance with this invention may be invasive ductal carcinoma, ductal carcinoma in situ, or invasive lobular carcinoma. Alternatively, BC may be classified on the basis of receptor status. In another embodiment, the breast cancer to be diagnosed, detected, monitored or screened in accordance with this invention may therefore be:

"normal" BC (ER+, PR+, HER2+, cytokeratin 5/6+, and HER1+),

"luminal A" BC (ER+ and/or PR+, HER2-),

"luminal B" BC (ER+ and/or PR+, HER2+),

"triple-negative" BC (ER-, PR-, HER2-),

"HER2+/ER-" BC (ER-, PR-, and HER2+), or

"unclassified BC" (ER-, PR-, HER2-, cytokeratin 5/6-, and HER1-).

In the method of the invention, the level of a miR gene product in the urine sample, relative to the level of the respective miR gene product in a control sample, is typically increased by at least 10% or at least 25% or at least 50% or at least 100%, to be indicative of breast cancer. The level of a miR gene product in the urine sample, relative to the level of the respective miR gene product in a control sample, is usually decreased by at least 10% or at least 25% or at least 50%, to be indicative of breast cancer.

In the method of the invention, the level of miR-660 gene product, and/or let7-i gene product in the urine sample from the subject, relative to the control level of the corresponding miR gene product, may be decreased by at least 25% to be indicative of breast cancer, and/or the level of miR-424 gene product, in the urine sample from the subject, relative to the control level of miR-424 gene product, may be increased by at least 50% to be indicative of breast cancer.

In another embodiment, the level of miR-660 gene product, and/or let7-i gene product in the urine sample from the subject, relative to the control level of the corrsponding miR gene product, may be decreased by at least 50% to be indicative of breast cancer, and/or the level of miR-424 gene product, in the urine sample from the subject, relative to the control level of miR-424 gene product, may be increased by at least 100% to be indicative of breast cancer.

In another embodiment, the level of miR-660 gene product, and/or let7-i gene product in the urine sample from the subject, relative to the control level of the corrsponding miR gene product, may be decreased by at least 75% to be indicative of breast cancer, and/or the level of miR-424 gene product, in the urine sample from the subject, relative to the control level of miR-424 gene product, may be increased by at least 200% to be indicative of breast cancer.

The subject may be diagnosed as having breast cancer if

- the level of miR-424 gene product in the urine sample from the subject is greater than 110%, greater than 125%, greater than 150%, or greater than 200% of the control level of miR-424 gene product (e.g. the level in the control sample);

- the level of miR-660 gene product in the urine sample from the subject is less than 90%, less than 75%, or less than 50% of the control level of miR-660 gene product (e.g. the level in the control sample); and

- the level of let7-i gene product in the urine sample from the subject is less than 90%, less than 75%, or less than 50% of the control level of let7-i gene product (e.g. the level in the control sample);
  and optionally if, in addition,

- the level of miR-423 gene product in the urine sample from the subject is less than 90%, less than 75%, or less than 50% of the control level of miR-423 gene product (e.g. the level in the control sample);

- the level of miR-125b gene product in the urine sample from the subject is greater than 110%, greater than 125%, greater than 150%, or greater than 200% of the control level of miR-125b gene product (e.g. the level in the control sample); and/or

- the level of let7-d gene product in the urine sample from the subject is greater than 110%, greater than 125%, greater than 150%, or greater than 200% of the control level of let7-d gene product (e.g. the level in the control sample).

In a particular embodiment, the subject is diagnosed as having breast cancer if

- the level of miR-424 gene product in the urine sample from the subject is greater than 110%, greater than 125%, greater than 150%, or greater than 200% of the control level of miR-424 gene product (e.g. the level in the control sample);

- the level of miR-660 gene product in the urine sample from the subject is less than 90%, less than 75%, or less than 50% of the control level of miR-660 gene product (e.g. the level in the control sample); and

- the level of let7-i gene product in the urine sample from the subject is less than 90%, less than 75%, or less than 50% of the control level of let7-i gene product (e.g. the level in the control sample);
  and optionally if, in addition,

- the level of miR-423 gene product in the urine sample from the subject is less than 90%, less than 75%, or less than 50% of the control level of miR-423 gene product (e.g. the level in the control sample);

- the level of miR-125b gene product in the urine sample from the subject is greater than 110%, greater than 125%, greater than 150%, or greater than 200% of the control level of miR-125b gene product (e.g. the level in the control sample); or

- the level of let7-d gene product in the urine sample from the subject is greater than 110%, greater than 125%, greater than 150%, or greater than 200% of the control level of let7-d gene product (e.g. the level in the control sample).

*Kits*

In another aspect, the invention pertains to use of a a diagnostic kit, or a microarray, comprising an oligonucleotide capable of hybridizing to a nucleic acid consisting of SEQ ID NO:11, an oligonucleotide capable of hybridizing to a nucleic acid consisting of SEQ ID NO:12, and an oligonucleotide capable of hybridizing to a nucleic acid consisting of SEQ ID NO:17, for the detection or diagnosis of breast cancer. The kit preferably further comprises an oligonucleotide capable of hybridizing to a nucleic acid consisting of SEQ ID NO:10, or an oligonucleotide capable of hybridizing to a nucleic acid consisting of SEQ ID NO:7, or an oligonucleotide capable of hybridizing to a nucleic acid consisting of SEQ ID NO:14.

As used herein, "capable of hybridizing" preferably refers to high stringency hybridization conditions. By "high stringency conditions" is meant that the nucleotide sequence specifically hybridizes to a target sequence (the nucleotide sequence of any of the nucleic acids described herein) in an amount that is detectably stronger than non-specific hybridization. High stringency conditions include conditions which would distinguish a polynucleotide with an exact complementary sequence from a random sequence that happened to have a few small regions (e.g., 3-10 bases) that matched the nucleotide sequence. Such small regions of complementarity are more easily melted than a full-length complement of 14-17 or more bases, and high stringency hybridization makes them easily distinguishable. High stringency conditions would include, for example, low salt and/or high temperature conditions, such as provided by 0.02-0.1 M NaCl, at temperatures of 50-70° C (e.g. 0.05 M NaCl at 60°C).

In yet another aspect, the invention relates to the use of an oligonucleotide capable of hybridizing to a nucleic acid consisting of SEQ ID NO:11, an oligonucleotide capable of hybridizing to a nucleic acid consisting of SEQ ID NO:12, and an oligonucleotide capable of hybridizing to a nucleic acid consisting of SEQ ID NO:17 for the diagnosis of breast cancer, said use comprising contacting said oligonucleotide(s) with a urine sample. The use preferably further comprises contacting an oligonucleotide capable of hybridizing to a nucleic acid consisting of SEQ ID NO:10, an oligonucleotide capable of hybridizing to a nucleic acid consisting of SEQ ID NO:7, or an oligonucleotide capable of hybridizing to a nucleic acid consisting of SEQ ID NO:14 with said urine sample. The oligonucleotides may be immobilized on a microarray.

In particular embodiments of the kit or use described above, the oligonucleotide capable of hybridizing to a nucleic acid consisting of SEQ ID NO:11 is perfectly complementary to SEQ ID NO:11, the oligonucleotide capable of hybridizing to a nucleic acid consisting of SEQ ID NO:12 is perfectly complementary to SEQ ID NO:12, and/or the oligonucleotide capable of hybridizing to a nucleic acid consisting of SEQ ID NO:17 is perfectly complementary to SEQ ID NO:17. Additionally, the oligonucleotide capable of hybridizing to a nucleic acid consisting of SEQ ID NO:10 may be perfectly complementary to SEQ ID NO:10. Additionally, the oligonucleotide capable of hybridizing to a nucleic acid consisting of SEQ ID NO:7 may be perfectly complementary to SEQ ID NO:7. Additionally, the oligonucleotide capable of hybridizing to a nucleic acid consisting of SEQ ID NO:14 may be perfectly complementary to SEQ ID NO:14. As used herein the term "complementary" refers to the reverse complement, unless indicated otherwise.

In other embodiments, the kit contains a pair of primers for amplifying a miR described herein. The kit may comprise a primer pair capable of amplifying miR-424, a primer pair capable of amplifying miR-660, and a primer pair capable of amplifying let7-i. The kit may further comprise a primer pair capable of amplifying miR-423, a primer pair capable of amplifying miR-125b, and/or a primer pair capable of amplifying let7-d. The kit may further comprise a primer pair capable of amplifying miR-17, a primer pair capable of amplifying let7-f, a primer pair capable of amplifying miR-222 and/or a primer pair capable of amplifying miR-194.

The kit may further comprise a universal primer for the reverse transcription reaction.

This diagnostic kit can be used for breast screening, e.g. by identifying changes in urine miRNA levels in breast cancer patients compared to normal cancer-free individuals, in a control group. The kit may further be used for the prognosis and/or prediction of outcome, e.g. by identifying differences between patients with early or late stage cancers, as well as stratifying patients into molecular subtypes. This information can then aid in strategic planning of an individual patients therapeutic regimen. Further, the kit may be used for the monitoring of response to treatments, e.g. through serial urinary miRNA measurements; particularly in the neoadjuvant chemotherapy and metastatic disease settings.

The methods, uses and kits of the invention have been described above in connection with breast cancer. The invention is also applicable for the diagnosis, detection and/or screening of other cancer types, e.g. of breast carcinomas, lung carcinomas, gastric carcinomas, esophageal carcinomas, colorectal carcinomas, liver carcinomas, ovarian carcinomas, thecomas, arrhenoblastomas, cervical carcinomas, endometrial carcinoma, endometrial hyperplasia, endometriosis, fibrosarcomas, choriocarcinoma, head and neck cancer, nasopharyngeal carcinoma, laryngeal carcinomas, hepatoblastoma, Kaposi's sarcoma, melanoma, skin carcinomas, hemangioma, cavernous hemangioma, hemangioblastoma, pancreas carcinomas, retinoblastoma, astrocytoma, glioblastoma, Schwannoma, oligodendroglioma, medulloblastoma, neuroblastomas, rhabdomyosarcoma, osteogenic sarcoma, leiomyosarcomas, urinary tract carcinomas, thyroid carcinomas, Wilm's tumor, renal cell carcinoma, prostate carcinoma, abnormal vascular proliferation associated with phakomatoses, edema (such as that associated with brain tumors), and/or Meigs' syndrome. The embodiments described above in connection with BC apply to theses other cancer types *mutatis mutandis.*

Another aspect of the present disclosure is a method for enriching or isolating extracellular vesicles, in particular exosomes, from a body fluid sample. The body fluid can be any body fluid, including, but not limited to, blood, plasma, urine, ascites, amniotic liquor, and saliva.

The method comprises filtrating the body fluid sample through a filter membrane which has protein binding properties. The filter membrane is preferably a polyamide membrane, e.g. a Nylon membrane. The pore size is typically from about 0.1 $\mu$m to about 1 $\mu$m, preferably from about 0.15 $\mu$m to about 500 $\mu$m, more preferably from about 0.18 $\mu$m to about 0.25 $\mu$m, e.g. about 0.22 $\mu$m.

In certain embodiments the method further comprises, prior to the filtration through the protein-binding membrane, a pre-filtration through a membrane which does not have protein-binding properties. The pre-filtration may serve to separate larger EVs, such as microvesicles, from exosomes which have a smaller size. Accordingly, the pore size of the pre-filtration membrane is typically from about 0.18 $\mu$m to about 0.25 $\mu$m, preferably from about 0.19 $\mu$m to about 0.21 $\mu$m, e.g. about 0.2 $\mu$m. The pre-filtration membrane may be made of a cellulosic material such as cellulose acetate.

Initially the body fluid sample may be centrifuged to remove cell debris.

After filtration through the protein-binding membrane the exosomes are bound to the membrane. The method of the invention further comprises recovering the exosomes from the membrane. This is typically achieved by contacting the membrane with a suitable solution such as a lysis buffer. Suitable buffers may include a buffer substance to maintain the pH of the lysis buffer in a range from about 6.5 to 8, or 7 to 7.5, e.g. about 7.2. The lysis buffer may contain a surfactant or detergent. Suitable surfactants include sodium lauryl sulfate and Triton X100.

The method for enriching or isolating exosomes or extracellular vesicles can be used in the method of diagnosing cancer described herein, e.g. for providing the urine sample.

The following examples illustrate the invention and should not be understood as limiting the invention to the exemplified embodiments.

**Example 1**

**1. Methods**

1.1 Cohort

The case-control cohort comprised of 69 untreated patients, newly diagnosed with primary BC in the adjuvant setting and 40 healthy female controls at the Department of Obstetrics and Gynecology, University Medical Center Freiburg. Control's health status was confirmed by medical examination performed by an experienced clinical physician including breast and regional lymph nodes to exclude potential BC and any history of other (malignant) disease or current inflammation. Furthermore healthy controls underwent breast and axillary ultrasound and mammography. By staging procedures according to the current national guidelines, all BC patients were tested to preclude cases of advanced stages with distant metastasis.

The according investigation protocols (36/12 and 386/16) were approved by the institutional ethical review board of the University of Freiburg. All patients and healthy controls involved provided written informed consent. Clinical cohort characteristics are summarized in Table 2.

Table 2: Cohort characteristics of breast cancer (BC) patients and healthy controls

| | BC patients | healthy controls | p value |
|---|---|---|---|
| N | 69 | 40 | |
| median age, y | 58 (30-79) | 45 (20-72) | 0. |
| **Histology** | | | |
| invasive ductal | 49 | | |
| invasive lobular | 5 | | |
| **Tumor stage** | | | |
| pT1a | 2 | | |
| pT1b | 5 | | |

(continued)

| | BC patients | healthy controls | p value |
|---|---|---|---|
| **Tumor stage** | | | |
| pT1c | 39 | | |
| pT2 | 17 | | |
| pT3 | 2 | | |
| **Nodal status** | | | |
| pN0 | 53 | | |
| pN1 | 11 | | |
| pN2 | 1 | | |
| **Grading** | | | |
| G1 | 10 | | |
| G2 | 43 | | |
| G3 | 8 | | |
| **Hormone receptor status** | | | |
| ER positive | 53 | | |
| PR positive | 50 | | |
| **HER2neu status** | | | |
| positive | 4 | | |

1.2 miRNA specimen

This biomarker identification study with diagnostic applicability in respect of breast cancer detection based on the expression analysis of urinary circulating miRNA types. The chosen set of thirteen miRNA specimen is characterized by the combined features of a proven functional relation to breast cancer and a robust and reliable detectability in human urine samples. Due to normalization purposes in relative quantification of miRNA expression levels two miRNA specimen with housekeeping characteristics (miR16, -26b) could be identified in a previous study [12]. Definite information on miRNA types including target sequences are listed in Table 2.

Table 2: Information on miRNA names and sequences utilized in expression analysis.

| miRNA | miRNA sequence | SEQ ID NO: |
|---|---|---|
| cel-miR39-3p[#] | UCACCGGGUGUAAAUCAGCUUG | 1 |
| ath-miR159a[#] | UUUGGAUUGAAGGGAGCUCUA | 2 |
| hS8-miR16-5p | UAGCAGCACGUAAAUAUUGGCG | 3 |
| hsa-miR17-5p | CAAAGUGCUUACAGUGCAGGUAG | 4 |
| hsa-miR26b-5p | UUCAAGUAAUUCAGGAUAGGU | 5 |
| hS8-miR107-5p | AGCAGCAUUGUACAGGGCUAUCA | 6 |
| hS8-miR125b-5p | UCCCUGAGACCCUAACUUGUGA | 7 |
| hS8-miR194-5p | UGUAACAGCAACUCCAUGUGGA | 8 |
| hS8-miR222-3p | AGCUACAUCUGGCUACUGGGU | 9 |
| hsa-miR423-5p | UGAGGGGCAGAGAGCGAGACUUU | 10 |
| hsa-miR424-5p | CAGCAGCAAUUCAUGUUUUGAA | 11 |
| hsa-miR660-5p | UACCCAUUGCAUAUCGGAGUUG | 12 |
| hsa-let7a-5p | UGAGGUAGUAGGUUGUAUAGUU | 13 |
| hsa-let7d-5p | AGAGGUAGUAGGUUGCAUAGUU | 14 |
| hsa-let7e-5p | UGAGGUAGGAGGUUGUAUAGUU | 15 |
| hsa-let7f-5p | UGAGGUAGUAGAUUGUAUAGUU | 16 |
| hsa-let7i-5p | UGAGGUAGUAGUUUGUGCUGUU | 17 |

[#]exogenous, synthetic spike-in control RNA specimen

1.3 Sampling and storage

Native spontaneous urine samples were collected in 100 ml sterile urine sampling cups (Sarstedt, Germany) and stored in 10 ml aliquots (Urin Monovette, Sarstedt, Germany) at - 80°C until further processing.

1.4 Sample preparation, RNA isolation and reverse transcription

Cryopreserved native urine specimen were thawed at room temperature and centrifuged (4000rpm; 15min; 4°C) to remove potential cell debris residues or other solid precipitates from pure urine supernatant.

For isolation of miRNA-loaded exosomes / microvesicles from urine samples, 10ml centrifuged urine were transferred to a 10ml syringe (#4617207V; BRAUN, Melsungen, Germany) with a screwed on 0.22µm nylon syringe filter (#02542904; Perkin Elmer, Waltham, USA). With piston inserted, the sample was filtered dropwise through the screwed-on filter. Following filtration, the filters were washed with 5ml DPBS buffer (#14190185; ThermoFisher Scientific, Karlsruhe, Germany) using a fresh 5ml syringe (#4617053V; BRAUN, Melsungen, Germany) to remove sample residues that possibly interfere with downstream applications. After washing, total RNA isolation was performed utilizing the Norgen Total RNA Purification Kit (#17200, NORGEN Biotek Corp., Thorold, Canada). According to manufacturer's protocol [58] (Appendix B: 'Total RNA Purification from Plasma or Serum') 600µl lysis buffer were extruded through filter membrane. Purified RNA was eluted in 35µl elution buffer. Further processing of RNA specimen was defined to a standard volume of 2.5µl.

Reverse transcription (RT) of miRNAs was realized by utilizing 2.5µl of RNA sample, 5µl of 5x RT-Buffer, 0.1mM of ATP, 0.5µM RT-primer, 0.1mM of each deoxynucleotide (dATP, dCTP, dGTP, dTTP), 25 units of Maxima Reverse Transcriptase (Thermo) and 1 unit of poly(A) polymerase (New England Biolabs GmbH, Frankfurt, Germany) in a final volume of 25µl. Reaction incubation was set to 30min at 42°C followed by enzyme inactivation at 85°C for 10min.

Primer design for RT reaction was geared to 'miRprimer' software tool by Busk [59]. RT Primer information is listed in Table 3. cDNA samples were diluted in H2O to a final volume of 200µl and stored at -20°C until further processing.

Table 3: Information on primers utilized in expression analysis of miRNA types.

| miRNA | sense primer | antisense primer |
|---|---|---|
| cel-miR39-3p# | GTCACCGGGTGTAAATCAG (SEQ ID NO:18) | GGTCCAGTTTTTTTTTTTTTTCAAG (SEQ ID NO:19) |
| ath-miR159a# | GCGCAGTTTGGATTGAAG (SEQ ID NO:20) | AGGTCCAGTTTTTTTTTTTTTTTAGAG (SEQ ID NO:21) |
| hsa-miR16-5p | CGCAGTAGCAGCACGTA (SEQ ID NO:22) | CAGTTTTTTTTTTTTTTTCGCCAA (SEQ ID NO:23) |
| hsa-miR17-5p | GCAAAGTGCTTACAGTGCAG (SEQ ID NO:24) | GGTCCAGTTTTTTTTTTTTTTTCTAC (SEQ ID NO:25) |
| hsa-miR26b-5p | CGCAGTTCAAGTAATTCAGGAT (SEQ ID NO:26) | GGTCCAGTTTTTTTTTTTTTTTACCT (SEQ ID NO:27) |
| hsa-miR107-5p | GCAGAGCAGCATTGTACAG (SEQ ID NO:28) | GGTCCAGTTTTTTTTTTTTTTTGATAG (SEQ ID NO:29) |
| hsa-miR125b-5p | GCAGTCCCTGAGACCCT (SEQ ID NO:30) | CCAGTTTTTTTTTTTTTTTCACAAGT (SEQ ID NO:31) |
| hsa-miR194-5p | CAGTGTAACAGCAACTCCA (SEQ ID NO:32) | TCCAGTTTTTTTTTTTTTTTCCACAT (SEQ ID NO:33) |
| hsa-miR222-3p | GCAGAGCTACATCTGGCT (SEQ ID NO:34) | CCAGTTTTTTTTTTTTTTTACCCAGT (SEQ ID NO:35) |
| hsa-miR423-5p | CAGTGAGGGGCAGAGAG (SEQ ID NO:36) | GGTCCAGTTTTTTTTTTTTTTAAAGTC (SEQ ID NO:37) |
| hsa-miR424-5p | AGCAGCAGCAATTCATGT (SEQ ID NO:38) | AGGTCCAGTTTTTTTTTTTTTTTCAA (SEQ ID NO:39) |
| hsa-miR660-5p | AGTACCCATTGCATATCGGA (SEQ ID NO:40) | GGTCCAGTTTTTTTTTTTTTTTCAAC (SEQ ID NO:41) |
| hsa-let7a-5p | GCAGTGAGGTAGTAGGTTG (SEQ ID NO:42) | GGTCCAGTTTTTTTTTTTTTTTAACTATAC (SEQ ID NO:43) |
| hsa-let7d-5p | CGCAGAGAGGTAGTAGGTTG (SEQ ID NO:44) | GGTCCAGTTTTTTTTTTTTTTTAACTATG (SEQ ID NO:45) |

(continued)

| miRNA | sense primer | antisense primer |
|-------|-------------|------------------|
| hsa-let7e-5p | GCAGTGAGGTAGGAGGTTG (SEQ ID NO: 46) | GGTCCAGTTTTTTTTTTTTTTTTAACTATAC (SEQ ID NO:47) |
| hsa-let7f-5p | CGCAGTGAGGTAGTAGATTG (SEQ ID NO: 48) | CAGGTCCAGTTTTTTTTTTTTTTTTAAC (SEQ ID NO:49) |
| hsa-let7i-5p | GCAGTGAGGTAGTAGTTTGTG (SEQ ID NO:50) | GGTCCAGTTTTTTTTTTTTTTTTTAACAG (SEQ ID NO:51) |
| miRNA RT* | CAGGTCCAGTTTTTTTTTTTTTTTVN (SEQ ID NO:52) | |

#exogenous, synthetic spike-in control RNA specimen; *universal primer for reverse transcription (RT) reaction.

## 1.5 qPCR-based miRNA quantification

Quantitative determination of miRNA expression levels was realized by real-time PCR on LightCycler® 480 (Roche, Mannheim, Germany). PCR reaction set up: 1µl cDNA, in-house qPCR buffer (containing TRIS pH8.1, dATP, dCTP, dGTP, dTTP, magnesium, potassium ammonium, SYBRGreen (Jena Bioscience, Jena, Germany), enhancers) 0.25U HotStart Taq Polymerase (Jena Bioscience) in a total volume of 10µl.

qPCR primers were designed via 'miRprimer' software [59] and are listed in Table 3.

PCR program comprised of initial denaturation (95°C; 2min); 40 cycles: denaturation (95°C; 5sec) / annealing/extension (60°C; 30sec); melting curve.

Relative quantification of miRNA expression levels was performed in a duplicate analysis based on ΔCt method normalized on corresponding mean expression values of housekeeping miRNAs miR-16 and -26b. Validation of housekeeping miRNAs applicable for the setting of urinary miRNA analysis was proven in our previous study [12] and is methodically described by Marabita et al. [60].

## 1.6 Technical processing normalization via spike-in control RNAs

Monitoring of processing efficiency in regard to RNA extraction, complementary DNA synthesis (reverse transcription) and PCR amplification was realized by addition of 'spike-in' control RNA specimen of defined concentration to the RNA lysis buffer as recommended by Marabita et al. [60]. Thereto, 5fM two exogenous synthetic RNA types *(Caenorhabditis elegans:* cel-miR-39 and *Arabidopsis thaliana:* ath-miR-159; Biomers.net GmbH, Ulm, Germany; sequences in Table 3) were added to RNA lysis buffer (Norgen).

Technical normalization of expression data obtained by qPCR was performed based on threshold points (Ct) with relative quantities (RQs) defined as those $C_t$ values scaled to the geometric mean of external reference spike-in RNAs and conversion to a linear scale RQ = $2^{-\Delta Ct}$ with $\Delta C_t = C_{t\ miRNA} - C_t$ spike geometric mean.

Normalization factor (NF) was calculated as geometric mean of the selected normalizers (housekeeper miRNA specimen (miR16, -26b) for each sample (*j*). Thus, the NF is applied to extract the normalized relative quantities (NRQ) for each miRNA i and *j* sample:

$$NRQij = RQij / NFj \ \ [60].$$

## 1.7 Statistical analyses

Biomarker assessment focused on the extraction of the diagnostic value of all thirteen analyzed miRNA types and combinations of them in regard to urine-based breast cancer detection. To this aim we used several statistical approaches, described as follows.

1. Variable selection frequencies with cross validation: In each of 10,000 repetitions, we randomly selected a subsample of size 2/3 of the data (the training sample), fitted a logistic regression model using forward selection and determined the inclusion frequency of each variable. We then chose the 7 variables with the highest inclusion frequency, fitted a model to the full data set and reduced this model further by forward selection, finally choosing within all convergent models the one that minimized the Akaike information criterion (AIC).

2. Variable selection frequencies with bootstrap: We drew 10,000 bootstrap samples (with repetition). In each bootstrap sample, we performed modelling and variable selection as in the first approach.

3. ROC analysis / cross validation: Similar to approach 1, but now we applied the model that was fitted in the training sample to the (remaining) test sample, with fixed coefficients (100 repetitions). We constructed the receiver operating characteristic (ROC) curves and used the area under the curve (AUC) as a measure of goodness of fit.

Variable selection frequencies with boosting: Boosting is a stepwise and regularized procedure for fitting generalized linear models. Starting with setting all regression coefficients to zero, in each step one coefficient is updated, such that the model fit improves most. The number of steps is determined by cross-validation. As many coefficients will never be updated, boosting provides a sparse model. The result was visualized by showing the coefficient paths. Boosting was performed using the R package GAMBoost [61].
4. All subsets regression: All possible $2^{12}$ = 4096 models were fitted and compared based on the AIC. We then restricted the list to convergent models with at most 4 variables.
All analyses were performed using the open statistical software environment R [62].

## 2. Results

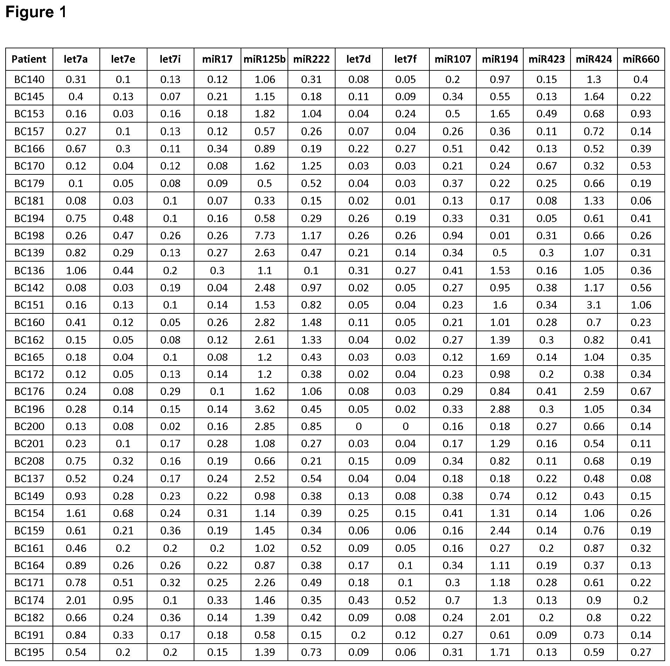

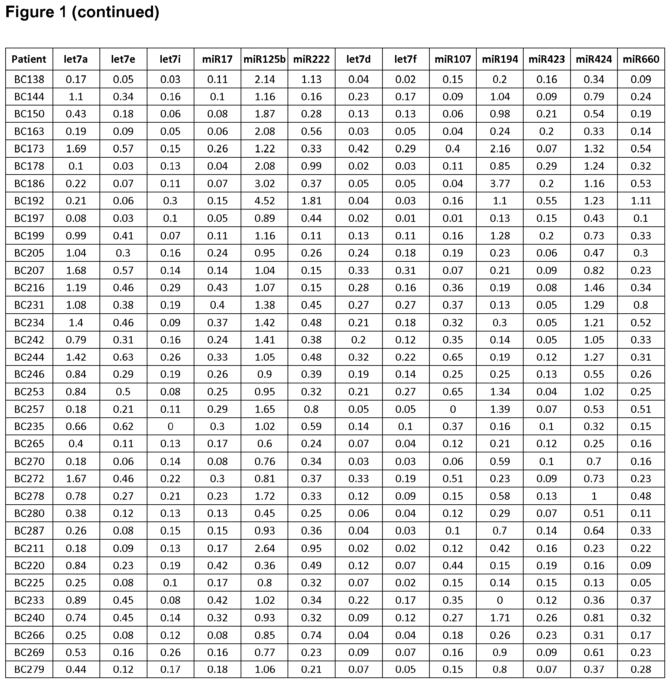

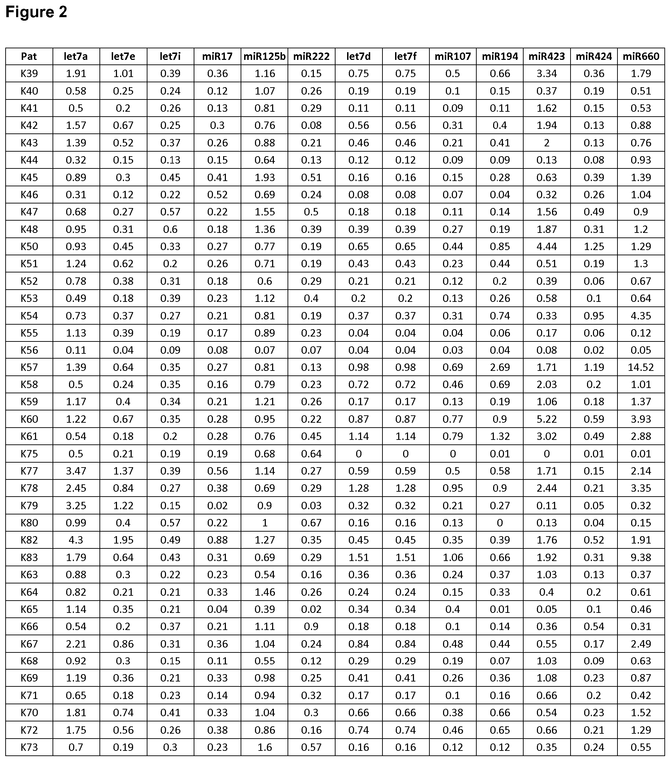

The complete panel of all thirteen circulating miRNA specimen could be reliably detected in all urine specimen of the test cohort. Sample-dependent variations in expression levels of miRNAs due to variable urine parameters (e.g. dilution, concentration of nucleic acids) could be equilibrated by normalization against the set of two stably expressed housekeeping miRNAs 16 and 26b.
Based on qPCR-derived miRNA expression levels converted to normalized relative quantities (NRQ; Figure 1 and Figure 2), the assessment of miRNA type-specific diagnostic power in regard to discrimination purposes of breast cancer cases against healthy controls was evaluated by employing several statistical methods (see Methods section). Thereby the applicability and validity of the methods were compared. These methods were considered in both, a singular fashion each and in a combined manner. The summarized information output of the utilized statistical analyses is described in the following (1)-(5).

(1) Variable selection frequencies with cross validation

The empirical cumulative distribution functions of the regression coefficients over all 10,000 repetitions resulted in the following ranking scheme in miRNA type frequencies (inclusion frequency, relating to all selected models, in brackets; up = upregulated expression, down = downregulated expression).

| | | | |
|---|---|---|---|
| 1. | miR-424 | (95.98%; up) |
| 2. | miR-423 | (72.3%; down) |
| 3. | miR-660 | (68.7%; down) |
| 4. | let7-i | (59.97%; down) |
| 5. | miR-17 | (15.96%; up) |
| 6. | let7-f | (14.65%; down) |
| 7. | miR-222 | (9.82%; up) |
| 8. | miR-194 | (8,59%; up) |
| 9. | miR-125b | (3.57%; up) |
| 10. | let7-d | (3.25%; down) |

The forward-selection based on these ten variables (miRNA types) led to a model including the first five variables (miR-424, miR-423, miR-660, let7-i, miR-17). Since models with more than four variables (miRNA types) failed to converge, these were excluded from further statistical assessment. Applying these restrictions, this statistical approach provided the model: **miR-424 + miR-423 + miR-660 + let7-i.**

(2) Variable selection frequencies with bootstrap

The empirical cumulative distribution functions of the regression coefficients over all 10,000 repetitions resulted in the following ranking of the most frequently chosen miRNA types was found.

| | | |
|---|---|---|
| 1. | miR-424 | (94.81%; up) |

(continued)

| | | |
|---|---|---|
| 2. | miR-660 | (64.23%; down) |
| 3. | miR-423 | (58.96%; down) |
| 4. | let7-i | (58.96%; down) |
| 5. | let7-f | (20.32%; down) |
| 6. | miR-17 | (12.46%; up) |
| 7. | miR-222 | (10.79%, up) |
| 8. | miR-194 | (9.44%; up) |
| 9. | miR-125b | (5.82%; up) |
| 10. | let7-d | (3.57%; up) |

Forward-selection led to a model with the five miRNA types let7-i, let7-f, miR-423, miR-424, miR-660. Again, the missing convergence of models with more than four variables led to the reduction to the following miRNA type combination, which is identical to the result of the first statistical approach: **miR-424 + miR-423 + miR-660 + let7-i.**

(3) ROC analysis / cross validation

The previously preferred model with four variables miR-424, miR-423, miR-660, let7-i (1, 2)) was selected in 18 of 100 repetitions. In all these repetitions the AUC was 1 both for the training sample and for the test sample, if the same model was newly fitted. When we applied the fitted model with fixed coefficients to the test sample, AUC values ranged between 0.905 and 1 with a median value of 0.995.
There was a number of alternative models with at most four miRNA types with promising AUC values. The most frequently selected combination of five miRNA types was again miR-424, miR-423, miR-660, let7-i, and miR-17.

(4) Variable selection frequencies with boosting

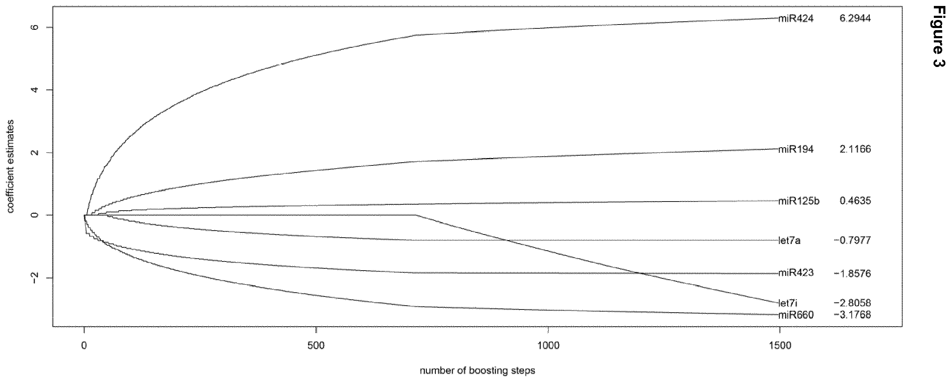

After 1500 boosting steps, seven miRNA types had been selected (including those obtained by the earlier approaches). These were miR-424 (up), miR-423 (down), miR-660 (down), let-7i (down), miR-194 (up), let7-a (down), miR-125b (up), see Figure 3.

(5) All subsets regression

All 4096 possible candidate models were fitted. The restriction to convergent models with at most four variables provided the following superior models:

| | | |
|---|---|---|
| 1. | miR-424 + miR-660 + let7-i + miR-125b | (AIC = 15.0) |
| 2. | miR-424 + mi-R423 + miR-660 + let7-i | (AIC = 16.3) |
| 3. | miR-424 + miR-660 + let7-i + let7-d | (AIC = 17.2) |
| 4. | miR-424 + miR-660 + let7-i | (AIC = 19.2) |

The first model characterized by the lowest AIC (AIC = 15.0) occurred only here, not in the previous approaches. The second model contains the four miRNA types (**miR-424 + miR-423 + miR-660 + let7-i**) with the highest inclusion frequency in approaches (1) and (2). The second model occurred also in approach (3).
Note that all subsets regression works without cross validation, as all models are fitted to the full data set. Thus, this regression method lacks information as regards the predictive performance of the models.
Summing up, a panel of four miRNA types, comprising **miR-424, miR-423, miR-660,** and **let7-i** could be elected as highly specific combinatory biomarker tool in discrimination of breast cancer patients vs. healthy controls based on urine specimen.

**Abbreviations**

| | |
|---|---|
| AIC | Akaike information criterion |
| AUC | area under the curve |

| miRNA / miR | micro ribonucleic acid |
| NF | normalization factor |
| NGS | next generation sequencing |
| NRQ | normalized relative quantities |
| PET | positron-emission tomography |
| qPCR | quantitative polymerase chain reaction |
| ROC | receiver operating characteristic |
| SNP | single nucleotide polymorphism |

**References**

1. Disease, G.B.D., I. Injury, and C. Prevalence, Global, regional, and national incidence, prevalence, and years lived with disability for 328 diseases and injuries for 195 countries, 1990-2016: a systematic analysis for the Global Burden of Disease Study 2016. Lancet, 2017. 390(10100): p. 1211-1259.

2. Sharma, R., Breast cancer incidence, mortality and mortality-to-incidence ratio (MIR) are associated with human development, 1990-2016: evidence from Global Burden of Disease Study 2016. Breast Cancer, 2019.

3. Momenimovahed, Z. and H. Salehiniya, Epidemiological characteristics of and risk factors for breast cancer in the world. Breast Cancer (Dove Med Press), 2019. 11: p. 151-164.

4. Oeffinger, K.C., et al., Breast Cancer Screening for Women at Average Risk: 2015 Guideline Update From the American Cancer Society. JAMA, 2015. 314(15): p. 1599-614.

5. Tabar, L., et al., Insights from the breast cancer screening trials: how screening affects the natural history of breast cancer and implications for evaluating service screening programs. Breast J, 2015. 21(1): p. 13-20.

6. Wockel, A., et al., Interdisciplinary Screening, Diagnosis, Therapy and Follow-up of Breast Cancer. Guideline of the DGGG and the DKG (S3-Level, AWMF Registry Number 032/045OL, December 2017) - Part 1 with Recommendations for the Screening, Diagnosis and Therapy of Breast Cancer. Geburtshilfe Frauenheilkd, 2018. 78(10): p. 927-948.

7. Sankaranarayanan, R., et al., Cancer survival in Africa, Asia, and Central America: a population-based study. Lancet Oncol, 2010. 11(2): p. 165-73.

8. Kolb, T.M., J. Lichy, and J.H. Newhouse, Comparison of the performance of screening mammography, physical examination, and breast US and evaluation of factors that influence them: an analysis of 27,825 patient evaluations. Radiology, 2002. 225(1): p. 165-75.

9. Wang, L., Early Diagnosis of Breast Cancer. Sensors (Basel), 2017. 17(7).

10. Arneth, B., Update on the types and usage of liquid biopsies in the clinical setting: a systematic review. BMC Cancer, 2018. 18(1): p. 527.

11. Salehi, M. and M. Sharifi, Exosomal miRNAs as novel cancer biomarkers: Challenges and opportunities. J Cell Physiol, 2018. 233(9): p. 6370-6380.

12. Erbes, T., et al., Feasibility of urinary microRNA detection in breast cancer patients and its potential as an innovative non-invasive biomarker. BMC Cancer, 2015. 15: p. 193.

13. Balacescu, O., et al., Urinary microRNAs for prostate cancer diagnosis, prognosis, and treatment response: are we there yet? Wiley Interdiscip Rev RNA, 2017. 8(6).

14. Gasparri, M.L., et al., Beyond circulating microRNA biomarkers: Urinary microRNAs in ovarian and breast cancer. Tumour Biol, 2017. 39(5): p. 1010428317695525.

15. Grayson, K., et al., Urine Biomarkers for the Early Detection of Ovarian Cancer - Are We There Yet? Biomark Cancer, 2019. 11: p. 1179299X19830977.

16. Franzen, C.A., et al., Urinary Exosomes: The Potential for Biomarker Utility, Intercellular Signaling and Therapeutics in Urological Malignancy. J Urol, 2016. 195(5): p. 1331-1339.

17. Balzeau, J., et al., The LIN28/let-7 Pathway in Cancer. Front Genet, 2017. 8: p. 31.

18. Dufresne, S., et al., A Review of Physical Activity and Circulating miRNA Expression: Implications in Cancer Risk and Progression. Cancer Epidemiol Biomarkers Prev, 2018. 27(1): p. 11-24.

19. Elghoroury, E.A., et al., Evaluation of miRNA-21 and miRNA Let-7 as Prognostic Markers in Patients With Breast Cancer. Clin Breast Cancer, 2018. 18(4): p. e721-e726.

20. Masood, N., et al., Entangling Relation of Micro RNA-let7, miRNA-200 and miRNA-125 with Various Cancers. Pathol Oncol Res, 2017. 23(4): p. 707-715.

21. Sen, C.K., et al., Micromanaging vascular biology: tiny microRNAs play big band. J Vasc Res, 2009. 46(6): p. 527-40.

22. Thammaiah, C.K. and S. Jayaram, Role of let-7 family microRNA in breast cancer. Noncoding RNA Res, 2016. 1(1): p. 77-82.

23. de Anda-Jauregui, G., et al., Nonredundant, Highly Connected MicroRNAs Control Functionality in Breast Cancer Networks. Int J Genomics, 2018. 2018: p. 9585383.

24. Lehmann, T.P., et al., Relative levels oflet-7a, miR-17, miR-27b, miR-125a, miR-125b and miR-206 as potential molecular markers to evaluate grade, receptor status and molecular type in breast cancer. Mol Med Rep, 2015. 12(3): p. 4692-4702.

25. Sun, H., et al., Let7 miRNAs sensitize breast cancer stem cells to radiationinduced repression through inhibition of the cyclin D1/Akt1/Wnt1 signaling pathway. Mol Med Rep, 2016. 14(4): p. 3285-92.

26. Bobbili, M.R., et al., OncomiR-17-5p: alarm signal in cancer? Oncotarget, 2017. 8(41): p. 71206-71222.

27. Hesari, A., et al., Expression of circulating miR-17, miR-25, and miR-133 in breast cancer patients. J Cell Biochem, 2018.

28. Li, J., et al., miR-17-5p suppresses cell proliferation and invasion by targeting ETV1 in triple- negative breast cancer. BMC Cancer, 2017. 17(1): p. 745.

29. Swellam, M., et al., Role of some circulating MiRNAs on breast cancer diagnosis. Arch Physiol Biochem, 2018: p. 1-9.

30. Wang, Y., et al., MiR-17-5p may serve as a novel predictor for breast cancer recurrence. Cancer Biomark, 2018. 22(4): p. 721-726.

31. Feliciano, A., et al., miR-125b acts as a tumor suppressor in breast tumorigenesis via its novel direct targets ENPEP, CK2-alpha, CCNJ, and MEGF9. PLoS One, 2013. 8(10): p. e76247.

32. Ferracin, M., et al., miR-125b targets erythropoietin and its receptor and their expression correlates with metastatic potential and ERBB2/HER2 expression. Mol Cancer, 2013. 12(1): p. 130.

33. Luo, Y., et al., Elevated microRNA-125b levels predict a worse prognosis in HER2-positive breast cancer patients. Oncol Lett, 2017. 13(2): p. 867-874.

34. Mar-Aguilar, F., et al., Differential expression of miR-21, miR-125b and miR-191 in breast cancer tissue. Asia Pac J Clin Oncol, 2013. 9(1): p. 53-9.

35. Amini, S., et al., Expression Analysis of MicroRNA-222 in Breast Cancer. Clin Lab, 2018. 64(4): p. 491-496.

36. Liu, S., et al., miR-221/222 activate the Wnt/beta-catenin signaling to promote triple-negative breast cancer. J Mol Cell Biol, 2018. 10(4): p. 302-315.

37. Liang, Y.K., et al., MiR-221/222 promote epithelial-mesenchymal transition by targeting Notch3 in breast cancer cell lines. NPJ Breast Cancer, 2018. 4: p. 20.

38. Han, S.H., et al., MicroRNA-222 Expression as a Predictive Marker for Tumor Progression in Hormone Receptor-Positive Breast Cancer. J Breast Cancer, 2017. 20(1): p. 35-44.

39. Ding, J., et al., Exosome-mediated miR-222 transferring: An insight into NF-kappaB-mediated breast cancer metastasis. Exp Cell Res, 2018. 369(1): p. 129-138.

40. Gao, B., et al., MicroRNA-107 is downregulated and having tumor suppressive effect in breast cancer by negatively regulating brain-derived neurotrophic factor. J Gene Med, 2017. 19(12).

41. Petrovic, N., et al., MicroRNA in breast cancer: The association with BRCA1/2. Cancer Biomark, 2017. 19(2): p. 119-128.

42. Ai, H., et al., microRNAs-107 inhibited autophagy, proliferation, and migration of breast cancer cells by targeting HMGB1. J Cell Biochem, 2018.

43. Zhang, L., et al., MiR-107 down-regulates SIAH1 expression in human breast cancer cells and silencing of miR-107 inhibits tumor growth in a nude mouse model of triple-negative breast cancer. Mol Carcinog, 2016. 55(5): p. 768-77.

44. Li, X.Y., et al., MiRNA-107 inhibits proliferation and migration by targeting CDK8 in breast cancer. Int J Clin Exp Med, 2014. 7(1): p. 32-40.

45. Kleivi Sahlberg, K., et al., A serum microRNA signature predicts tumor relapse and survival in triple-negative breast cancer patients. Clin Cancer Res, 2015. 21(5): p. 1207-14.

46. Stuckrath, I., et al., Aberrant plasma levels of circulating miR-16, miR-107, miR-130a and miR-146a are associated with lymph node metastasis and receptor status of breast cancer patients. Oncotarget, 2015. 6(15): p. 13387-401.

47. Yang, F., Z. Xiao, and S. Zhang, Knockdown of miR-194-5p inhibits cell proliferation, migration and invasion in breast cancer by regulating the Wnt/beta-catenin signaling pathway. Int J Mol Med, 2018. 42(6): p. 3355-3363.

48. Huo, D., et al., Identification of a circulating microRNA signature to distinguish recurrence in breast cancer patients. Oncotarget, 2016. 7(34): p. 55231-55248.

49. Le, X.F., et al., Modulation of MicroRNA-194 and cell migration by HER2-targeting trastuzumab in breast cancer. PLoS One, 2012. 7(7): p. e41170.

50. Zhao, H., et al., Genetic analysis and preliminary function study of miR-423 in breast cancer. Tumour Biol, 2015. 36(6): p. 4763-71.

51. Smith, R.A., et al., A genetic variant located in miR-423 is associated with reduced breast cancer risk. Cancer Genomics Proteomics, 2012. 9(3): p. 115-8.

52. Murria Estal, R., et al., MicroRNA signatures in hereditary breast cancer. Breast Cancer Res Treat, 2013. 142(1): p. 19-30.

53. Wang, J., et al., miR-424-5p regulates cell proliferation, migration and invasion by targeting doublecortin-like kinase 1 in basal-like breast cancer. Biomed Pharmacother, 2018. 102: p. 147-152.

54. Rodriguez-Barrueco, R., et al., miR-424(322)/503 is a breast cancer tumor suppressor whose loss promotes resistance to chemotherapy. Genes Dev, 2017. 31(6): p. 553-566.

55. Zhang, L., et al., A circulating miRNA signature as a diagnostic biomarker for non-invasive early detection of breast cancer. Breast Cancer Res Treat, 2015. 154(2): p. 423-34.

56. Shen, Y., et al., Inhibition of miR-660-5p expression suppresses tumor development and metastasis in human breast cancer. Genet Mol Res, 2017. 16(1).

57. Krishnan, P., et al., Next generation sequencing profiling identifies miR-574-3p and miR-660-5p as potential novel prognostic markers for breast cancer. BMC Genomics, 2015. 16: p. 735.

58. Norgen-Biotek-Corp. Total RNA Purification Kit Product Insert Product # 17200, 37500, 17250 https://norqenbiotek.com/sites/default/files/resources/Total%20RNA%20Purification%20Kit%    20Insert%20PI17200-33.pdf. 2018.

59. Busk, P.K., A tool for design of primers for microRNA-specific quantitative RT-qPCR. BMC Bioinformatics, 2014. 15: p. 29.

60. Marabita, F., et al., Normalization of circulating microRNA expression data obtained by quantitative real-time RT-PCR. Brief Bioinform, 2016. 17(2): p. 204-12.

61. Tutz, G. and H. Binder, Generalized additive modeling with implicit variable selection by likelihood-based boosting. Biometrics, 2006. 62(4): p. 961-71.

62. R-Core-Team, R: A language and environment for statistical computing https.-//www.r-proiect.org/. 2018.

63. Ghosh, R.K., T. Pandey, and P. Dey, Liquid biopsy: A new avenue in pathology. Cytopathology, 2019. 30(2): p. 138-143.

64. Mader, S. and K. Pantel, Liquid Biopsy: Current Status and Future Perspectives. Oncol Res Treat, 2017. 40(7-8): p. 404-408.

65. Pantel, K. and C. Alix-Panabieres, Liquid biopsy and minimal residual disease - latest advances and implications for cure. Nat Rev Clin Oncol, 2019. 16(7): p. 409-424.

66. Rossi, G. and M. Ignatiadis, Promises and Pitfalls of Using Liquid Biopsy for Precision Medicine. Cancer Res, 2019. 79(11): p. 2798-2804.

67. Alimirzaie, S., M. Bagherzadeh, and M.R. Akbari, Liquid biopsy in breast cancer: A comprehensive review. Clin Genet, 2019. 95(6): p. 643-660.

68. Wang, H.X. and G. Olivier, Tumor-derived extracellular vesicles in breast cancer: from bench to bedside. Cancer Lett, 2019.

69. Zubor, P., et al., Why the Gold Standard Approach by Mammography Demands Extension by Multiomics? Application of Liquid Biopsy miRNA Profiles to Breast Cancer Disease Management. Int J Mol Sci, 2019. 20(12).

70. Beretov, J., et al., Proteomic Analysis of Urine to Identify Breast Cancer Biomarker Candidates Using a Label-Free LC-MS/MS Approach. PLoS One, 2015. 10(11): p. e0141876.

71. Burton, C., et al., Urinary metallomics as a novel biomarker discovery platform: Breast cancer as a case study. Clin Chim Acta, 2016. 452: p. 142-8.

72. Guo, C., et al., Discriminating patients with early-stage breast cancer from benign lesions by detection of oxidative DNA damage biomarker in urine. Oncotarget, 2017. 8(32): p. 53100-53109.

73. Herman-Saffar, O., et al., Early non-invasive detection of breast cancer using exhaled breath and urine analysis.

Comput Biol Med, 2018. 96: p. 227-232.

74. Woollam, M., et al., Detection of Volatile Organic Compounds (VOCs) in Urine via Gas Chromatography-Mass Spectrometry QTOF to Differentiate Between Localized and Metastatic Models of Breast Cancer. Sci Rep, 2019. 9(1): p. 2526.

75. Hofbauer, S. L., et al., A urinary microRNA (miR) signature for diagnosis of bladder cancer. Urol Oncol, 2018. 36(12): p. 531 e1-531 e8.

76. Kutwin, P., et al., Urine miRNA as a potential biomarker for bladder cancer detection - a meta-analysis. Cent European J Urol, 2018. 71(2): p. 177-185.

77. Lekchnov, E.A., et al., Searching for the Novel Specific Predictors of Prostate Cancer in Urine: The Analysis of 84 miRNA Expression. Int J Mol Sci, 2018. 19(12).

78. Moldovan, L., et al., Methodological challenges in utilizing miRNAs as circulating biomarkers. J Cell Mol Med, 2014. 18(3): p. 371-90.

79. Liu, J., et al., Tumour-suppressive microRNA-424-5p directly targets CCNE1 as potential prognostic markers in epithelial ovarian cancer. Cell Cycle, 2018. 17(3): p. 309-318.

80. Wei, S., et al., miR-424-5p promotes proliferation of gastric cancer by targeting Smad3 through TGF-beta signaling pathway. Oncotarget, 2016. 7(46): p. 75185-75196.

81. Wu, K., et al., MicroRNA-424-5p suppresses the expression of SOCS6 in pancreatic cancer. Pathol Oncol Res, 2013. 19(4): p. 739-48.

82. Xu, J., et al., Suppressed miR-424 expression via upregulation of target gene Chk1 contributes to the progression of cervical cancer. Oncogene, 2013. 32(8): p. 976-87.

83. Xie, D., et al., MicroRNA424 serves an antioncogenic role by targeting cyclindependent kinase 1 in breast cancer cells. Oncol Rep, 2018. 40(6): p. 3416-3426.

84. Shivapurkar, N., et al., Circulating microRNAs in patients with hormone receptor-positive, metastatic breast cancer treated with dovitinib. Clin Transl Med, 2017. 6(1): p. 37.

85. Fortunato, O., et al., Mir-660 is downregulated in lung cancer patients and its replacement inhibits lung tumorigenesis by targeting MDM2-p53 interaction. Cell Death Dis, 2014. 5: p. e1564.

86. He, T., et al., miR6605p is associated with cell migration, invasion, proliferation and apoptosis in renal cell carcinoma. Mol Med Rep, 2018. 17(1): p. 2051-2060.

87. He, X. and Y. Shu, RNA N6-methyladenosine modification participates in miR-660/E2F3 axis- mediated inhibition of cell proliferation in gastric cancer. Pathol Res Pract, 2019. 215(6): p. 152393.

88. Borzi, C., et al., mir-660-p53-mir-486 Network: A New Key Regulatory Pathway in Lung Tumorigenesis. Int J Mol Sci, 2017. 18(1).

89. Zhang, P., et al., Downregulation of microRNA660 inhibits cell proliferation and invasion in osteosarcoma by directly targeting forkhead box O1. Mol Med Rep, 2018. 18(2): p. 2433-2440.

90. Zhou, C., et al., Combination of serum miRNAs with Cyfra21-1 for the diagnosis of non-small cell lung cancer. Cancer Lett, 2015. 367(2): p. 138-46.

91. Elkhadragy, L., et al., A regulatory BMI1/let-7i/ERK3 pathway controls the motility of head and neck cancer cells. Mol Oncol, 2017. 11(2): p. 194-207.

92. Gadducci, A., et al., Micro-RNAs and ovarian cancer: the state of art and perspectives of clinical research.

Gynecol Endocrinol, 2014. 30(4): p. 266-71.

93. Hur, K., et al., Identification of a metastasis-specific MicroRNA signature in human colorectal cancer. J Natl Cancer Inst, 2015. 107(3).

94. Qin, M.M., et al., let-7i inhibits proliferation and migration of bladder cancer cells by targeting HMGA1. BMC Urol, 2019. 19(1): p. 53.

95. Xie, J., et al., miR-7 inhibits the invasion and metastasis of gastric cancer cells by suppressing epidermal growth factor receptor expression. Oncol Rep, 2014. 31(4): p. 1715-22.

96. Yang, W.H., et al., Repression of bone morphogenetic protein 4 by let-7i attenuates mesenchymal migration of head and neck cancer cells. Biochem Biophys Res Commun, 2013. 433(1): p. 24-30.

97. Mahn, R., et al., Circulating microRNAs (miRNA) in serum of patients with prostate cancer. Urology, 2011. 77(5): p. 1265 e9-16.

98. Xiao, D., et al., Melanoma cell-derived exosomes promote epithelial-mesenchymal transition in primary melanocytes through paracrine/autocrine signaling in the tumor microenvironment. Cancer Lett, 2016. 376(2): p. 318-27.

99. Chakraborty, C. and S. Das, Profiling cell-free and circulating miRNA: a clinical diagnostic tool for different cancers. Tumour Biol, 2016. 37(5): p. 5705-14.

100. Zhu, Y., et al., Identification of a serum microRNA expression signature for detection of lung cancer, involving miR-23b, miR-221, miR-148b and miR-423-3p. Lung Cancer, 2017. 114: p. 6-11.

101. Du, W., Z. Feng, and Q. Sun, LncRNA LINC00319 accelerates ovarian cancer progression through miR-423-5p/NACC1 pathway. Biochem Biophys Res Commun, 2018. 507(1-4): p. 198-202.

102. Lin, H., et al., Inhibition of miR-423-5p suppressed prostate cancer through targeting GRIM-19. Gene, 2019. 688: p. 93-97.

103. Yang, H., et al., Exosomal miR-423-5p targets SUFU to promote cancer growth and metastasis and serves as a novel marker for gastric cancer. Mol Carcinog, 2018. 57(9): p. 1223-1236.

104. McDermott, A.M., et al., Identification and validation of oncologic miRNA biomarkers for luminal A-like breast cancer. PLoS One, 2014. 9(1): p. e87032.

105. Ding, H.X., et al., MiRNA Polymorphisms and Cancer Prognosis: A Systematic Review and Meta-Analysis. Front Oncol, 2018. 8: p. 596.

106. Campos-Parra, A.D., et al., Micro-RNAs as Potential Predictors of Response to Breast Cancer Systemic Therapy: Future Clinical Implications. Int J Mol Sci, 2017. 18(6).

107. Liu, B., et al., Serum miR-21 and miR-125b as markers predicting neoadjuvant chemotherapy response and prognosis in stage II/III breast cancer. Hum Pathol, 2017. 64: p. 44-52.

108. Xie, X., et al., The role of miR-125b-mitochondria-caspase-3 pathway in doxorubicin resistance and therapy in human breast cancer. Tumour Biol, 2015. 36(9): p. 7185-94.

109. Hong, L., et al., miR-125b inhibited epithelial-mesenchymal transition of triple-negative breast cancer by targeting MAP2K7. Onco Targets Ther, 2016. 9: p. 2639-48.

110. Matamala, N., et al., Tumor microRNA expression profiling identifies circulating microRNAs for early breast cancer detection. Clin Chem, 2015. 61(8): p. 1098-106.

111. Das, R., et al., MicroRNA-194 Promotes Prostate Cancer Metastasis by Inhibiting SOCS2. Cancer Res, 2017. 77(4): p. 1021-1034.

112. Kong, Q., et al., MicroRNA-194 suppresses prostate cancer migration and invasion by downregulating human nuclear distribution protein. Oncol Rep, 2017. 37(2): p. 803-812.

113. Peng, Y., et al., MiRNA-194 activates the Wnt/beta-catenin signaling pathway in gastric cancer by targeting the negative Wnt regulator, SUFU. Cancer Lett, 2017. 385: p. 117-127.

114. Zhang, X., et al., MicroRNA-194 represses glioma cell epithelialtomesenchymal transition by targeting Bmi1. Oncol Rep, 2017. 37(3): p. 1593-1600.

115. Chen, Y., et al., Promotional effect of microRNA-194 on breast cancer cells via targeting F- box/WD repeat-containing protein 7. Oncol Lett, 2018. 15(4): p. 4439-4444.

116. Gilles, M.E. and F.J. Slack, Let-7 microRNA as a potential therapeutic target with implications for immunotherapy. Expert Opin Ther Targets, 2018. 22(11): p. 929-939.

117. Chernyi, V.S., et al., Search of MicroRNAs Regulating the Receptor Status of Breast Cancer In Silico and Experimental Confirmation of Their Expression in Tumors. Bull Exp Biol Med, 2017. 163(5): p. 655-659.

118. Sueta, A., et al., Differential expression of exosomal miRNAs between breast cancer patients with and without recurrence. Oncotarget, 2017. 8(41): p. 69934-69944.

119. Zavesky, L., et al., Supernatant versus exosomal urinary microRNAs. Two fractions with different outcomes in gynaecological cancers. Neoplasma, 2016. 63(1): p. 121-32.

120. Armand-Labit, V. and A. Pradines, Circulating cell-free microRNAs as clinical cancer biomarkers. Biomol Concepts, 2017. 8(2): p. 61-81.

121. Nedaeinia, R., et al., Circulating exosomes and exosomal microRNAs as biomarkers in gastrointestinal cancer. Cancer Gene Ther, 2017. 24(2): p. 48-56.

122. Pritchard, C.C., et al., Blood cell origin of circulating microRNAs: a cautionary note for cancer biomarker studies. Cancer Prev Res (Phila), 2012. 5(3): p. 492-497.

123. Zhang, J., et al., Exosome and exosomal microRNA: trafficking, sorting, and function. Genomics Proteomics Bioinformatics, 2015. 13(1): p. 17-24.

124. Larssen, P., et al., Tracing Cellular Origin of Human Exosomes Using Multiplex Proximity Extension Assays. Mol Cell Proteomics, 2017. 16(3): p. 502-511.

**Example 2- only urine samples are according to the invention**

**Materials and Methods**

*Specimen origin*

Human body fluids including serum, urine, ascites, and amniotic liquor served as matrices to test the filter-based microvesicle isolation method. The entirety of human specimen samples used in this study, originate from surplus material of either routine medical interventions or voluntary donators.

*Specimen pre-treatment and storage conditions*

Liquid biopsy specimen were obtained according to the WMA declaration of Helsinki [30]. In general, body fluid specimen were collected and stored in sterile sampling tubes at -20°C until further processing. Additional pre-treatment steps and detailed information are described for each sample matrix in the following.

*Serum:* Blood samples were collected by using S-Monovette Serum Gel (#04.1925, Sarstedt AG, Nuembrecht, Germany) and allowed to clot at room temperature for 20min followed by centrifugation at 2500g for 10min at 20°C to obtain pure serum. Serum samples were transferred to a fresh 15 ml poly propylene (pp) tube (#62.554.502; Sarstedt AG) and stored at -20°C until further processing.

*Urine:* Patients provided urine specimen collected in a sterile 70ml screw-on cap urine sampling tube (#75.9922.745, Sarsted AG). Urine samples were transferred and aliquoted to 10ml Urine Monovette (#10.252; Sarsted AG) and stored at -20°C until further processing.

**Microvesicle isolation**

*Pre-treatment following storage*

Fresh frozen liquid biopsy samples were thawed at room temperature and centrifuged at 4000g for 15min to remove sediment and cellular debris. Following centrifugation, supernatant was transferred in a new 15ml pp tube (Sarstedt AG) for further processing.

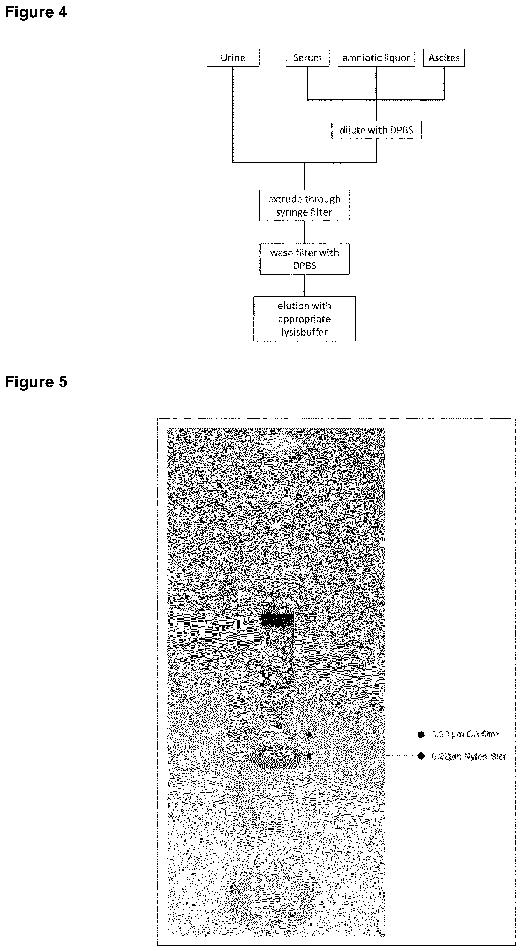

Due to the higher viscosity and protein content, the specimen matrices serum, ascites and amniotic liquor were diluted with sterile 1x Dulbecco's phosphate-buffered saline (DPBS, #14190185; ThermoFisher Scientific, Karlsruhe, Germany) in ratio 2:3 (2ml sample:3ml DPBS) to facilitate the filtration process. Sample matrix-dependent methodical variations in workflow are visualized in Figure 4.

*Arrangement of equipment and general processing*

Pre-treated sample liquids were transferred individually to a 20ml syringe (#4617207V; BRAUN, Melsungen, Germany) with a screwed on $0.2\mu m$ cellulose acetate (CA) syringe filter (#514-0061; VWR International GmbH, Darmstadt, Germany) followed by a second screwed on $0.22\mu m$ nylon syringe filter (#02542904; Perkin Elmer, Waltham, USA). Upon insertion of the piston, the individual samples were filtered dropwise through the screwed on filters (Fig. 5). Following filtration of the complete sample volume, the cascaded filters were washed with 5ml 1x DPBS (Thermo) using a fresh 5ml syringe (#4617053V; BRAUN, Melsungen, Germany). Washing guarantees the removal of sample residues falling below preset filter limitation that possibly interfere with downstream applications. Rinsed filters were then separated.

The CA filter retained all particles >200nm, including microvesicles within the respective molecular size limit. Due to the non-protein binding characteristics of the upstream CA filter, all microvesicles <200nm flow through and subsequently bound on the protein binding nylon membrane of the second filter.

Elution of the two different filter-bound microvesicle fractions was carried out by extruding $500\mu l$ protein lysis buffer (100mM HEPES pH 7.2, 0.1% SDS, 0.1% TRITON X100) through each of the used separate filters with a new 2ml syringe.

*Sample matrix-specific processing*

For isolation of urinary microvesicles 20ml centrifuged urine/sample were utilized in the filtration step with satisfying yield independent from inter-sample concentration differences.

Serum, ascites and amniotic liquor specimen were diluted with DPBS as fore cited and a final volume of 5ml was filtrated to harvest the two different size fractions of microvesicles.

**Western blot**

Protein concentrations of specimen isolates were quantified utilizing BCA method (#23227; ThermoFisher Scientific, Karlsruhe, Germany). Protein extracts obtained via the novel isolation method were analyzed by Western blot technique. $10\mu g$ of total protein were separated by SDS-PAGE and transferred to an IMMOBILON-P membrane (#IPVH00010; Merck, Darmstadt, Germany) by using a Mini PROTEAN tetra cell (BioRad, München, Germany). Following transfer, membranes were blocked with 3% skim milk in PBS-T and subsequently incubated with the primary antibody (AnnexinV #8555, Flotillin1 #18634S, HSP70 # 46477S, CD9 # 13174S CellSignaling Technology, $\beta$-Actin # SC-69879 Santa Cruz Biotechnology) over night at 4°C.

Primary antibody was rinsed by two washing steps with PBS-T, before membranes were incubated with the secondary antibody (anti-rabbit HRP # 7074S, CellSignaling Technology, anti-mouse HRP # 115-036-003, Jackson ImmunoResearch Europe) for 1 hour at room temperature. Incubation with secondary antibody was stopped by triplicate membrane washing with PBS-T.

ECL reaction was carried out according to Haan & Behrmann [31]. X-Ray films (Super RX-N, Fujifilm Europe, Düsseldorf, Germany) were processed utilizing the AGFA CP1000 X-Ray film processor.

**Results**

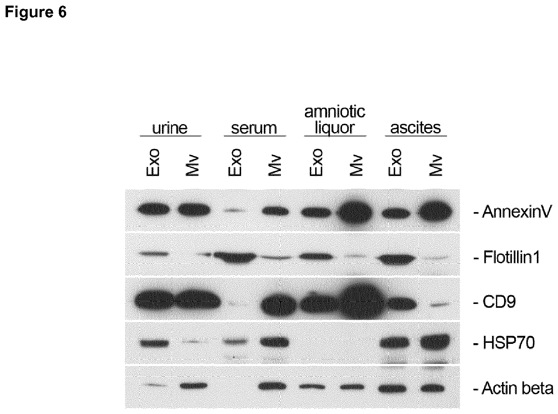

The results are depicted in the Western Blot shown in Figure 6.

References only for Example 2:

1. Kreger, B.T., et al., Microvesicle Cargo and Function Changes upon Induction of Cellular Transformation. J Biol Chem, 2016. 291(38): p. 19774-85.

2. Tricarico, C., J. Clancy, and C. D'Souza-Schorey, Biology and biogenesis of shed microvesicles. Small GTPases, 2017. 8(4): p. 220-232.

3. Colombo, M., G. Raposo, and C. Thery, Biogenesis, secretion, and intercellular interactions of exosomes and other extracellular vesicles. Annu Rev Cell Dev Biol, 2014. 30: p. 255-89.

4. Bakhshandeh, B., M.A. Kamaleddin, and K. Aalishah, A Comprehensive Review on Exosomes and Microvesicles as Epigenetic Factors. Curr Stem Cell Res Ther, 2017. 12(1): p. 31-36.

5. Vitkova, V., J. Zivny, and J. Janota, Endothelial cell-derived microvesicles: potential mediators and biomarkers of pathologic processes. Biomark Med, 2018. 12(2): p. 161-175.

6. van der Pol, E., et al., Recent developments in the nomenclature, presence, isolation, detection and clinical impact of extracellular vesicles. J Thromb Haemost, 2016. 14(1): p. 48-56.

7. Kowal, J., et al., Proteomic comparison defines novel markers to characterize heterogeneous populations of extracellular vesicle subtypes. Proc Natl Acad Sci U S A, 2016. 113(8): p. E968-77.

8. Cocucci, E. and J. Meldolesi, Ectosomes and exosomes: shedding the confusion between extracellular vesicles. Trends Cell Biol, 2015. 25(6): p. 364-72.

9. Minciacchi, V.R., M.R. Freeman, and D. Di Vizio, Extracellular vesicles in cancer: exosomes, microvesicles and the emerging role of large oncosomes. Semin Cell Dev Biol, 2015. 40: p. 41-51.

10. Gopal, S.K., et al., Extracellular vesicles: their role in cancer biology and epithelial-mesenchymal transition. Biochem J, 2017. 474(1): p. 21-45.

11. Xu, R., et al., Extracellular vesicle isolation and characterization: toward clinical application. J Clin Invest, 2016. 126(4): p. 1152-62.

12. Chen, Y., et al., Extracellular vesicles as novel biomarkers and pharmaceutic targets of diseases. Acta Pharmacol Sin, 2018. 39(4): p. 499-500.

13. Valencia, K. and F. Lecanda, Microvesicles: Isolation, Characterization for In Vitro and In Vivo Procedures. Methods Mol Biol, 2016. 1372: p. 181-92.

14. Erdbrugger, U. and J. Lannigan, Analytical challenges of extracellular vesicle detection: A comparison of different techniques. Cytometry A, 2016. 89(2): p. 123-34.

15. Choi, D.S., et al., Proteomics of extracellular vesicles: Exosomes and ectosomes. Mass Spectrom Rev, 2015. 34(4): p. 474-90.

16. Choi, D.S., et al., Proteomics, transcriptomics and lipidomics of exosomes and ectosomes. Proteomics, 2013. 13(10-11): p. 1554-71.

17. Enderle, D., et al., Characterization of RNA from Exosomes and Other Extracellular Vesicles Isolated by a Novel Spin Column-Based Method. PLoS One, 2015. 10(8): p. e0136133.

18. Chiriaco, M.S., et al., Lab-on-Chip for Exosomes and Microvesicles Detection and Characterization. Sensors (Basel), 2018. 18(10).

19. Szatanek, R., et al., Isolation of extracellular vesicles: Determining the correct approach (Review). Int J Mol Med, 2015. 36(1): p. 11-7.

20. Yuana, Y., et al., Handling and storage of human body fluids for analysis of extracellular vesicles. J Extracell Vesicles, 2015. 4: p. 29260.

21. Giebel, B. and C. Helmbrecht, Methods to Analyze EVs. Methods Mol Biol, 2017. 1545: p. 1-20.

22. Keerthikumar, S., et al., Bioinformatics Tools for Extracellular Vesicles Research. Methods Mol Biol, 2017. 1545: p. 189-196.

23. Yuana, Y., A. Sturk, and R. Nieuwland, Extracellular vesicles in physiological and pathological conditions. Blood Rev, 2013. 27(1): p. 31-9.

24. Boing, A.N., et al., Single-step isolation of extracellular vesicles by size-exclusion chromatography. J Extracell Vesicles, 2014. 3.

25. Coumans, F.A., et al., Reproducible extracellular vesicle size and concentration determination with tunable resistive pulse sensing. J Extracell Vesicles, 2014. 3: p. 25922.

26. Gray, W.D., A.J. Mitchell, and C.D. Searles, An accurate, precise method for general labeling of extracellular vesicles. MethodsX, 2015. 2: p. 360-7.

27. Mrvar-Brecko, A., et al., Isolated microvesicles from peripheral blood and body fluids as observed by scanning electron microscope. Blood Cells Mol Dis, 2010. 44(4): p. 307-12.

28. Sun, Y., et al., Facile preparation of salivary extracellular vesicles for cancer proteomics. Sci Rep, 2016. 6: p. 24669.

29. Yuana, Y., et al., Co-isolation of extracellular vesicles and high-density lipoproteins using density gradient ultracentrifugation. J Extracell Vesicles, 2014. 3.

30. The World Medical Association, I. WMA Declaration of Helsinki - Ethical Principles for Medical Research Involving Human Subjects 2018 [cited 2018; Available from: https://www.wma.net/policies-post/wma-declaration-of-helsinki-ethical-principles-for-medical-research-involving-human-subjects/.

31. Haan, C. and I. Behrmann, A cost effective non-commercial ECL-solution for Western blot detections yielding strong signals and low background. J Immunol Methods, 2007. 318(1-2): p. 11-9.

## Claims

1. A method of diagnosing whether a subject has cancer, comprising determining the level of miR-424, miR-660 and let7-i in a urine sample, wherein an alteration in the level of miR-424, miR-660 and let7-i in the urine sample, relative to the level of the respective miR gene products in a control sample, is indicative of the subject having cancer.

2. The method of claim 1, further comprising determining the level of miR-423, miR-125b or let7-d in the urine sample, wherein an alteration in the level of miR-423, miR-125b or let7-d in the urine sample, relative to the level of the respective miR gene products in the control sample, is indicative of the subject having cancer.

3. The method of claim 1 or 2, further comprising determining the level of let7-f, miR-222, miR-194 and/or miR-17 in the urine sample from the subject, wherein an alteration in the level of let7-f, miR-222, miR-194 and/or miR-17 in the urine sample, relative to the level of the respective miR gene products in the control sample, is indicative of the subject having cancer.

4. The method of claim 1, comprising

   a) determining in the urine sample from the subject the level of miR-424, miR-660 and let7-i,
   b) comparing the level of miR-424 in the urine sample to a control level of miR-424, comparing the level of miR-660 in the urine sample to a control level of miR-660, and comparing the level of let7-i in the urine sample to a control level of let7-i; and
   c) diagnosing whether the subject has cancer (e.g. breast cancer), wherein (i) a decrease in the level of miR-660 in the urine sample, relative to the control level of miR-660, (ii) a decrease in the level of let7-i in the urine sample, relative to the control level of let7-i, and (iii) an increase in the level of miR-424 in the urine sample, relative to the control level of miR-424, is indicative of the subject having cancer (e.g. breast cancer).

5. The method of claim 4, further comprising:

   a) determining in the urine sample from the subject the level of miR-423, miR-125b or let7-d,
   b) comparing the level of miR-423, miR-125b or let7-d in the urine sample to the control level of the respective miR gene product, and
   c) diagnosing whether the subject has cancer (e.g. breast cancer), wherein an increase in the level of miR-125b or let7-d in the urine sample, relative to the respective control level of miR-125b or let7-d, or a decrease in the level of miR-423 in the urine sample, relative to the control level of miR-423, is indicative of the subject having cancer (e.g. breast cancer).

6. The method of claim 4 or 5, further comprising:

   a) determining in the urine sample from the subject the level of miR-17, let7-f, miR-222 and/or miR-194,
   b) comparing the level of miR-17, let7-f, miR-222 and/or miR-194 in the urine sample to a control level of the respectibe miR gene product, and
   c) diagnosing whether the subject has cancer (e.g. breast cancer), wherein an increase in the level of miR-17, miR-222 and/or miR-194 in the urine sample, relative to the respective control level of miR-17, miR-222 or miR-194, and/or a decrease in the level of let7-f, relative to the control level of let7-f, is indicative of the subject having cancer (e.g. breast cancer).

7. The method of any one of the preceding claims, wherein the control sample is a urine sample from one or more healthy individuals.

8. The method of any one of the preceding claims, wherein the subject is a female human.

9. The method of any one of the preceding claims, wherein the level of miR gene product(s) is measured by quantitative RT-PCR, digital PCR, next generation sequencing, or by hybridisation using an oligonucleotide microarray.

10. The method of any one of the preceding claims, wherein the levels of miR-660 and let7-i in the urine sample from the subject, relative to the respective control levels of the respective gene products, are decreased by at least 25% to be indicative of breast cancer, and/or wherein the level of miR-424, in the urine sample from the subject, relative to the control level of miR-424 gene product, is increased by at least 25% to be indicative of breast cancer.

11. The use of a kit for the detection or diagnosis of breast cancer, said kit comprising an oligonucleotide capable of hybridizing to a nucleic acid consisting of SEQ ID NO:11, an oligonucleotide capable of hybridizing to a nucleic acid consisting of SEQ ID NO:12, and an oligonucleotide capable of hybridizing to a nucleic acid consisting of SEQ ID NO:17; optionally further comprising further comprising an oligonucleotide capable of hybridizing to a nucleic acid consisting of SEQ ID NO:7, an oligonucleotide capable of hybridizing to a nucleic acid consisting of SEQ ID NO:10 and/or an oligonucleotide capable of hybridizing to a nucleic acid consisting of SEQ ID NO:14.

12. The use of an oligonucleotide capable of hybridizing to a nucleic acid consisting of SEQ ID NO:11, of an oligonucleotide capable of hybridizing to a nucleic acid consisting of SEQ ID NO:12 and of an oligonucleotide capable of hybridizing to a nucleic acid consisting of SEQ ID NO:17 for the diagnosis of breast cancer, said use comprising contacting the oligonucleotide(s) with a urine sample, wherein said use optionally further comprises contacting an oligonucleotide capable of hybridizing to a nucleic acid consisting of SEQ ID NO:7, an oligonucleotide capable of hybridizing to a nucleic acid consisting of SEQ ID NO:10, and/or an oligonucleotide capable of hybridizing to a nucleic acid consisting of SEQ ID NO:14 with said urine sample.

13. The method of any one of claims 1 to 10, wherein the urine sample is provided by enriching or isolating extracellular vesicles or exosomes by a method comprising (i) optionally filtrating urine through a first membrane, wherein said first membrane has a pore size from 180 nm to 250 nm and does not bind proteins; (ii) filtrating the urine or, if step (i) is carried out, the filtrate obtained from step (i) through a second membrane, wherein said second membrane has a pore size from 180 nm to 250 nm and is a protein-binding membrane; and (iii) recovering the extracellular vesicles or exosomes from the second membrane.

14. The method of claim 13, wherein the second membrane has a pore size of about 0.22 $\mu$m and comprises, or substantially consists of, a polyamide.

**Patentansprüche**

1. Verfahren zur Diagnose, ob ein Individuum Krebs hat, umfassend das Bestimmen des Spiegels von miR-424, miR-660 und let7-i in einer Urinprobe, wobei eine Veränderung des Spiegels von miR-424, miR-660 und let7-i in der Urinprobe, bezogen auf den Spiegel der jeweiligen miR-Genprodukte in einer Kontrollprobe, angibt, dass das Individuum Krebs hat.

2. Verfahren nach Anspruch 1, ferner umfassend das Bestimmen des Spiegels von miR-423, miR-125b oder let7-d in der Urinprobe, wobei eine Veränderung des Spiegels von miR-423, miR-125b oder let7-d in der Urinprobe, bezogen auf den Spiegel der jeweiligen miR-Genprodukte in einer Kontrollprobe, angibt, dass das Individuum Krebs hat.

3. Verfahren nach Anspruch 1 oder 2, ferner umfassend das Bestimmen des Spiegels von let7-f, miR-222, miR-194 und/oder miR-17 in der Urinprobe von dem Individuum, wobei eine Veränderung des Spiegels von let7-f, miR-222, miR-194 und/oder miR-17 in der Urinprobe, bezogen auf den Spiegel der jeweiligen miR-Genprodukte in der Kontrollprobe, angibt, dass das Individuum Krebs hat.

4. Verfahren nach Anspruch 1, umfassend

    a) Bestimmen des Spiegels von miR-424, miR-660 und let7-i in der Urinprobe von dem Individuum,
    b) Vergleichen des Spiegels von miR-424 in der Urinprobe mit einem Kontrollspiegel von miR-424, Vergleichen des Spiegels von miR-660 in der Urinprobe mit einem Kontrollspiegel von miR-660 und Vergleichen des Spiegels von let7-i in der Urinprobe mit einem Kontrollspiegel von let7-i; und
    c) Diagnostizieren, ob das Individuum Krebs hat (z. B. Brustkrebs), wobei (i) eine Senkung des Spiegels von miR-660 in der Urinprobe, bezogen auf den Kontrollspiegel von miR-660, (ii) eine Senkung des Spiegels von let7-i in der Urinprobe, bezogen auf den Kontrollspiegel von let7-i, und (iii) eine Steigerung des Spiegels von miR-424 in der Urinprobe, bezogen auf den Kontrollspiegel von miR-424, angibt, dass das Individuum Krebs hat (z. B. Brustkrebs).

5. Verfahren nach Anspruch 4, ferner umfassend:

a) Bestimmen des Spiegels von miR-423, miR-125b oder let7-d in der Urinprobe von dem Individuum,
b) Vergleichen des Spiegels von miR-423, miR-125b oder let7-d in der Urinprobe mit dem Kontrollspiegel von dem jeweiligen miR-Genprodukt und
c) Diagnostizieren, ob das Individuum Krebs hat (z. B. Brustkrebs), wobei eine Steigerung des Spiegels von miR-125b oder let7-d in der Urinprobe, bezogen auf den jeweiligen Kontrollspiegel von miR-125b oder let7-d, oder eine Senkung des Spiegels von miR-423 in der Urinprobe, bezogen auf den Kontrollspiegel von miR-423, angibt, dass das Individuum Krebs hat (z. B. Brustkrebs).

6. Verfahren nach Anspruch 4 oder 5, ferner umfassend:

a) Bestimmen des Spiegels von miR-17, let7-f, miR-222 und/oder miR-194 in der Urinprobe von dem Individuum,
b) Vergleichen des Spiegels von miR-17, let7-f, miR-222 und/oder miR-194 in der Urinprobe mit einem Kontrollspiegel von dem jeweiligen miR-Genprodukt, und
c) Diagnostizieren, ob das Individuum Krebs hat (z. B. Brustkrebs), wobei eine Steigerung des Spiegels von miR-17, miR-222 und/oder miR-194 in der Urinprobe, bezogen auf den jeweiligen Kontrollspiegel von miR-17, miR-222 oder miR-194, und/oder eine Senkung des Spiegels von let7-f, bezogen auf den Kontrollspiegel von let7-f, angibt, dass das Individuum Krebs hat (z. B. Brustkrebs).

7. Verfahren nach einem der vorstehenden Ansprüche, wobei die Kontrollprobe eine Urinprobe von einem gesunden Individuum oder mehreren gesunden Individuen ist.

8. Verfahren nach einem der vorstehenden Ansprüche, wobei das Individuum eine Frau ist.

9. Verfahren nach einem der vorstehenden Ansprüche, wobei der Spiegel von miR-Genprodukt(en) mittels quantitativer RT-PCR, digitaler PCR, Sequenzierung der nächsten Generation oder Hybridisierung unter Verwendung eines Oligonucleotid-Mikroarrays gemessen wird.

10. Verfahren nach einem der vorstehenden Ansprüche, wobei die Spiegel von miR-660 und let7-i in der Urinprobe von dem Individuum, bezogen auf die jeweiligen Kontrollspiegel von den jeweiligen Genprodukten, um mindestens 25 % gesenkt sind, wenn sie Brustkrebs angeben, und/oder wobei der Spiegel von miR-424 in der Urinprobe von dem Individuum, bezogen auf den Kontrollspiegel des miR-424-Genproduktes, um mindestens 25 % gesteigert ist, wenn er Brustkrebs angibt.

11. Verwendung eines Kits für den Nachweis oder die Diagnose von Brustkrebs, wobei der Kit ein Oligonucleotid, das an eine Nukleinsäure, bestehend aus SEQ ID NO:11, hybridisieren kann, ein Oligonucleotid, das an eine Nukleinsäure, bestehend aus SEQ ID NO:12, hybridisieren kann, und ein Oligonucleotid, das an eine Nukleinsäure, bestehend aus SEQ ID NO:17, hybridisieren kann, umfasst; gegebenenfalls ferner umfassend ein Oligonucleotid, das an eine Nukleinsäure, bestehend aus SEQ ID NO:7, hybridisieren kann, ein Oligonucleotid, das an eine Nukleinsäure, bestehend aus SEQ ID NO:10, hybridisieren kann, und/oder ein Oligonucleotid, das an eine Nukleinsäure, bestehend aus SEQ ID NO:14, hybridisieren kann.

12. Verwendung eines Oligonucleotids, das an eine Nukleinsäure, bestehend aus SEQ ID NO:11, hybridisieren kann, eines Oligonucleotids, das an eine Nukleinsäure, bestehend aus SEQ ID NO:12, hybridisieren kann, und eines Oligonucleotids, das an eine Nukleinsäure, bestehend aus SEQ ID NO:17, hybridisieren kann, zur Diagnose von Brustkrebs, wobei die Verwendung das Kontaktieren des Oligonucleotids/der Oligonucleotide mit einer Urinprobe umfasst, wobei die Verwendung gegebenenfalls ferner das Kontaktieren eines Oligonucleotids, das an eine Nukleinsäure, bestehend aus SEQ ID NO:7, hybridisieren kann, eines Oligonucleotids, das an eine Nukleinsäure, bestehend aus SEQ ID NO:10, hybridisieren kann, und/oder eines Oligonucleotids, das an eine Nukleinsäure, bestehend aus SEQ ID NO:14, hybridisieren kann, mit der Urinprobe umfasst.

13. Verfahren nach einem der Ansprüche 1 bis 10, wobei die Urinprobe durch Anreichern oder Isolieren extrazellulärer Vesikel oder Exosome mittels eines Verfahrens, umfassend (i) gegebenenfalls Filtrieren von Urin durch eine erste Membran, wobei die erste Membran eine Porengröße von 180 nm bis 250 nm hat und keine Proteine bindet; (ii) Filtrieren des Urins oder, bei der Durchführung von Schritt (i), des aus Schritt (i) erhaltenen Filtrats durch eine zweite Membran, wobei die zweite Membran eine Porengröße von 180 nm bis 250 nm hat und eine proteinbindende Membran ist; und (iii) Gewinnen der extrazellulären Vesikel oder Exosome aus der zweiten Membran, bereitgestellt wird.

14. Verfahren nach Anspruch 13, wobei die zweite Membran eine Porengröße von etwa 0,22 $\mu$m hat und ein Polyamid umfasst oder im Wesentlichen daraus besteht.

**Revendications**

1. Procédé pour le diagnostic du cancer chez un sujet, comprenant la détermination du niveau de miR-424, miR-660 et let7-i dans un échantillon d'urine, dans lequel une altération du niveau de miR-424, miR-660 et let7-i dans l'échantillon d'urine, par rapport au niveau des produits génétiques miR respectifs dans un échantillon de contrôle, indique que le sujet est atteint d'un cancer.

2. Procédé selon la revendication 1, comprenant en outre la détermination du niveau de miR-423, miR-125b ou let7-d dans l'échantillon d'urine, dans lequel une altération du niveau de miR-423, miR-125b ou let7-d dans l'échantillon d'urine, par rapport au niveau des produits génétiques miR respectifs dans l'échantillon de contrôle, indique que le sujet est atteint d'un cancer.

3. Procédé selon la revendication 1 ou 2, comprenant en outre la détermination du niveau de let7-f, miR-222, miR-194 et/ou miR-17 dans l'échantillon d'urine du sujet, dans lequel une altération du niveau de let7-f, miR-222, miR-194 et/ou miR-17 dans l'échantillon d'urine, par rapport au niveau des produits génétiques miR respectifs dans l'échantillon de contrôle, indique que le sujet est atteint d'un cancer.

4. Procédé selon la revendication 1, comprenant

   a) déterminer dans l'échantillon d'urine du sujet le niveau de miR-424, miR-660 et let7-i,
   b) comparer le niveau de miR-424 dans l'échantillon d'urine à un niveau de contrôle de miR-424, comparer le niveau de miR-660 dans l'échantillon d'urine à un niveau de contrôle de miR-660, et comparer le niveau de let7-i dans l'échantillon d'urine à un niveau de contrôle de let7-i ; et
   c) diagnostiquer si le sujet a un cancer (par exemple un cancer du sein), dans lequel (i) une diminution du niveau de miR-660 dans l'échantillon d'urine, par rapport au niveau de contrôle de miR-660, (ii) une diminution du niveau de let7-i dans l'échantillon d'urine, par rapport au niveau de contrôle de let7-i, et (iii) une augmentation du niveau de miR-424 dans l'échantillon d'urine, par rapport au niveau de contrôle de miR-424, est une indication que le sujet a un cancer (par exemple un cancer du sein).

5. Procédé selon la revendication 4, comprenant en outre :

   a) déterminer dans l'échantillon d'urine du sujet le niveau de miR-423, miR-125b ou let7-d,
   b) comparer le niveau de miR-423, miR-125b ou let7-d dans l'échantillon d'urine au niveau de contrôle du produit génétique miR respectif, et
   c) diagnostiquer si le sujet a un cancer (par exemple un cancer du sein), dans lequel une augmentation du niveau de miR-125b ou de let7-d dans l'échantillon d'urine, par rapport au niveau de contrôle respectif de miR-125b ou de let7-d, ou une diminution du niveau de miR-423 dans l'échantillon d'urine, par rapport au niveau de contrôle de miR-423, indiquant que le sujet a un cancer (par exemple un cancer du sein).

6. Procédé selon la revendication 4 ou 5, comprenant en outre les étapes consistant à :

   a) déterminer dans l'échantillon d'urine du sujet le niveau de miR-17, let7-f, miR-222 et/ou miR-194,
   b) comparer le niveau de miR-17, let7-f, miR-222 et/ou miR-194 dans l'échantillon d'urine à un niveau de contrôle du produit génétique miR respectif, et
   c) diagnostiquer si le sujet a un cancer (par exemple un cancer du sein), une augmentation du niveau de miR-17, miR-222 et/ou miR-194 dans l'échantillon d'urine, par rapport au niveau de contrôle respectif de miR-17, miR-222 ou miR-194, et/ou une diminution du niveau de let7-f, par rapport au niveau de contrôle de let7-f, étant une indication que le sujet a un cancer (par exemple un cancer du sein).

**7.** Procédé selon l'une quelconque des revendications précédentes, dans lequel l'échantillon de contrôle est un échantillon d'urine provenant d'un ou de plusieurs individus sains.

**8.** Procédé selon l'une quelconque des revendications précédentes, dans lequel le sujet est une femme.

**9.** Procédé selon l'une quelconque des revendications précédentes, dans lequel le niveau de produit(s) génétique(s) miR est mesuré par RT-PCR quantitative, PCR numérique, séquençage de nouvelle génération ou par hybridation à l'aide d'un microréseau d'oligonucléotides.

**10.** Procédé selon l'une quelconque des revendications précédentes, dans lequel les niveaux de miR-660 et de let7-i dans l'échantillon d'urine du sujet, par rapport aux niveaux de contrôle respectifs des produits génétiques respectifs, sont diminués d'au moins 25 % pour indiquer un cancer du sein, et/ou dans lequel le niveau de miR-424, dans l'échantillon d'urine du sujet, par rapport au niveau de contrôle du produit génétique miR-424, est augmenté d'au moins 25 % pour indiquer un cancer du sein.

**11.** Utilisation d'un kit pour la détection ou le diagnostic du cancer du sein, ledit kit comprenant un oligonucléotide capable de s'hybrider à un acide nucléique constitué de SEQ ID NO : 11, un oligonucléotide capable de s'hybrider à un acide nucléique constitué de SEQ ID NO : 12, et un oligonucléotide capable de s'hybrider à un acide nucléique constitué de SEQ ID NO : 17 ; comprenant éventuellement en outre un oligonucléotide capable de s'hybrider à un acide nucléique constitué de SEQ ID NO : 7, un oligonucléotide capable de s'hybrider à un acide nucléique constitué de SEQ ID NO : 10 et/ou un oligonucléotide capable de s'hybrider à un acide nucléique constitué de SEQ ID NO : 14.

**12.** Utilisation d'un oligonucléotide capable de s'hybrider à un acide nucléique constitué de SEQ ID NO : 12, d'un oligonucléotide capable de s'hybrider à un acide nucléique constitué de SEQ ID NO : 12 et d'un oligonucléotide capable de s'hybrider à un acide nucléique constitué de SEQ ID NO : 17 pour le diagnostic du cancer du sein, ladite utilisation consistant à mettre en contact le ou les oligonucléotides avec un échantillon d'urine, ladite utilisation consistant en outre à mettre en contact un oligonucléotide capable de s'hybrider à un acide nucléique constitué de SEQ ID NO : 7, un oligonucléotide capable de s'hybrider à un acide nucléique constitué de SEQ ID NO : 10, et/ou un oligonucléotide capable de s'hybrider à un acide nucléique constitué de SEQ ID NO : 14, avec ledit échantillon d'urine.

**13.** Procédé selon l'une quelconque des revendications 1 à 10, dans lequel l'échantillon d'urine est pourvu de l'enrichissement ou l'isolement de vésicules extracellulaires ou d'exosomes par un procédé comprenant (i) la filtration éventuelle de l'urine à travers une première membrane, dans lequel ladite première membrane a une taille de pore comprise entre 180 nm et 250 nm et ne lie pas les protéines ; (ii) la filtration de l'urine ou, si l'étape i) est réalisée, le filtrat obtenu à l'étape (i) à travers une seconde membrane, dans lequel ladite seconde membrane ayant une taille de pore comprise entre 180 nm et 250 nm et étant une membrane liant les protéines ; et iii) la récupération des vésicules extracellulaires ou les exosomes à partir de la seconde membrane.

**14.** Procédé selon la revendication 13, dans lequel la seconde membrane a une taille de pore d'environ 0,22 $\mu$m et comprend, ou est substantiellement constituée, d'un polyamide.

## Figure 1

| Patient | let7a | let7e | let7i | miR17 | miR125b | miR222 | let7d | let7f | miR107 | miR194 | miR423 | miR424 | miR660 |
|---|---|---|---|---|---|---|---|---|---|---|---|---|---|
| BC140 | 0.31 | 0.1 | 0.13 | 0.12 | 1.06 | 0.31 | 0.08 | 0.05 | 0.2 | 0.97 | 0.15 | 1.3 | 0.4 |
| BC145 | 0.4 | 0.13 | 0.07 | 0.21 | 1.15 | 0.18 | 0.11 | 0.09 | 0.34 | 0.55 | 0.13 | 1.64 | 0.22 |
| BC153 | 0.16 | 0.03 | 0.16 | 0.18 | 1.82 | 1.04 | 0.04 | 0.24 | 0.5 | 1.65 | 0.49 | 0.68 | 0.93 |
| BC157 | 0.27 | 0.1 | 0.13 | 0.12 | 0.57 | 0.26 | 0.07 | 0.04 | 0.26 | 0.36 | 0.11 | 0.72 | 0.14 |
| BC166 | 0.67 | 0.3 | 0.11 | 0.34 | 0.89 | 0.19 | 0.22 | 0.27 | 0.51 | 0.42 | 0.13 | 0.52 | 0.39 |
| BC170 | 0.12 | 0.04 | 0.12 | 0.08 | 1.62 | 1.25 | 0.03 | 0.03 | 0.21 | 0.24 | 0.67 | 0.32 | 0.53 |
| BC179 | 0.1 | 0.05 | 0.08 | 0.09 | 0.5 | 0.52 | 0.04 | 0.03 | 0.37 | 0.22 | 0.25 | 0.66 | 0.19 |
| BC181 | 0.08 | 0.03 | 0.1 | 0.07 | 0.33 | 0.15 | 0.02 | 0.01 | 0.13 | 0.17 | 0.08 | 1.33 | 0.06 |
| BC194 | 0.75 | 0.48 | 0.1 | 0.16 | 0.58 | 0.29 | 0.26 | 0.19 | 0.33 | 0.31 | 0.05 | 0.61 | 0.41 |
| BC198 | 0.26 | 0.47 | 0.26 | 0.26 | 7.73 | 1.17 | 0.26 | 0.26 | 0.94 | 0.01 | 0.31 | 0.66 | 0.26 |
| BC139 | 0.82 | 0.29 | 0.13 | 0.27 | 2.63 | 0.47 | 0.21 | 0.14 | 0.34 | 0.5 | 0.3 | 1.07 | 0.31 |
| BC136 | 1.06 | 0.44 | 0.2 | 0.3 | 1.1 | 0.1 | 0.31 | 0.27 | 0.41 | 1.53 | 0.16 | 1.05 | 0.36 |
| BC142 | 0.08 | 0.03 | 0.19 | 0.04 | 2.48 | 0.97 | 0.02 | 0.05 | 0.27 | 0.95 | 0.38 | 1.17 | 0.56 |
| BC151 | 0.16 | 0.13 | 0.1 | 0.14 | 1.53 | 0.82 | 0.05 | 0.04 | 0.23 | 1.6 | 0.34 | 3.1 | 1.06 |
| BC160 | 0.41 | 0.12 | 0.05 | 0.26 | 2.82 | 1.48 | 0.11 | 0.05 | 0.21 | 1.01 | 0.28 | 0.7 | 0.23 |
| BC162 | 0.15 | 0.05 | 0.08 | 0.12 | 2.61 | 1.33 | 0.04 | 0.02 | 0.27 | 1.39 | 0.3 | 0.82 | 0.41 |
| BC165 | 0.18 | 0.04 | 0.1 | 0.08 | 1.2 | 0.43 | 0.03 | 0.03 | 0.12 | 1.69 | 0.14 | 1.04 | 0.35 |
| BC172 | 0.12 | 0.05 | 0.13 | 0.14 | 1.2 | 0.38 | 0.02 | 0.04 | 0.23 | 0.98 | 0.2 | 0.38 | 0.34 |
| BC176 | 0.24 | 0.08 | 0.29 | 0.1 | 1.62 | 1.06 | 0.08 | 0.03 | 0.29 | 0.84 | 0.41 | 2.59 | 0.67 |
| BC196 | 0.28 | 0.14 | 0.15 | 0.14 | 3.62 | 0.45 | 0.05 | 0.02 | 0.33 | 2.88 | 0.3 | 1.05 | 0.34 |
| BC200 | 0.13 | 0.08 | 0.02 | 0.16 | 2.85 | 0.85 | 0 | 0 | 0.16 | 0.18 | 0.27 | 0.66 | 0.14 |
| BC201 | 0.23 | 0.1 | 0.17 | 0.28 | 1.08 | 0.27 | 0.03 | 0.04 | 0.17 | 1.29 | 0.16 | 0.54 | 0.11 |
| BC208 | 0.75 | 0.32 | 0.16 | 0.19 | 0.66 | 0.21 | 0.15 | 0.09 | 0.34 | 0.82 | 0.11 | 0.68 | 0.19 |
| BC137 | 0.52 | 0.24 | 0.17 | 0.24 | 2.52 | 0.54 | 0.04 | 0.04 | 0.18 | 0.18 | 0.22 | 0.48 | 0.08 |
| BC149 | 0.93 | 0.28 | 0.23 | 0.22 | 0.98 | 0.38 | 0.13 | 0.08 | 0.38 | 0.74 | 0.12 | 0.43 | 0.15 |
| BC154 | 1.61 | 0.68 | 0.24 | 0.31 | 1.14 | 0.39 | 0.25 | 0.15 | 0.41 | 1.31 | 0.14 | 1.06 | 0.26 |
| BC159 | 0.61 | 0.21 | 0.36 | 0.19 | 1.45 | 0.34 | 0.06 | 0.06 | 0.16 | 2.44 | 0.14 | 0.76 | 0.19 |
| BC161 | 0.46 | 0.2 | 0.2 | 0.2 | 1.02 | 0.52 | 0.09 | 0.05 | 0.16 | 0.27 | 0.2 | 0.87 | 0.32 |
| BC164 | 0.89 | 0.26 | 0.26 | 0.22 | 0.87 | 0.38 | 0.17 | 0.1 | 0.34 | 1.11 | 0.19 | 0.37 | 0.13 |
| BC171 | 0.78 | 0.51 | 0.32 | 0.25 | 2.26 | 0.49 | 0.18 | 0.1 | 0.3 | 1.18 | 0.28 | 0.61 | 0.22 |
| BC174 | 2.01 | 0.95 | 0.1 | 0.33 | 1.46 | 0.35 | 0.43 | 0.52 | 0.7 | 1.3 | 0.13 | 0.9 | 0.2 |
| BC182 | 0.66 | 0.24 | 0.36 | 0.14 | 1.39 | 0.42 | 0.09 | 0.08 | 0.24 | 2.01 | 0.2 | 0.8 | 0.22 |
| BC191 | 0.84 | 0.33 | 0.17 | 0.18 | 0.58 | 0.15 | 0.2 | 0.12 | 0.27 | 0.61 | 0.09 | 0.73 | 0.14 |
| BC195 | 0.54 | 0.2 | 0.2 | 0.15 | 1.39 | 0.73 | 0.09 | 0.06 | 0.31 | 1.71 | 0.13 | 0.59 | 0.27 |

**Figure 1 (continued)**

| Patient | let7a | let7e | let7i | miR17 | miR125b | miR222 | let7d | let7f | miR107 | miR194 | miR423 | miR424 | miR660 |
|---------|-------|-------|-------|-------|---------|--------|-------|-------|--------|--------|--------|--------|--------|
| BC138 | 0.17 | 0.05 | 0.03 | 0.11 | 2.14 | 1.13 | 0.04 | 0.02 | 0.15 | 0.2 | 0.16 | 0.34 | 0.09 |
| BC144 | 1.1 | 0.34 | 0.16 | 0.1 | 1.16 | 0.16 | 0.23 | 0.17 | 0.09 | 1.04 | 0.09 | 0.79 | 0.24 |
| BC150 | 0.43 | 0.18 | 0.06 | 0.08 | 1.87 | 0.28 | 0.13 | 0.13 | 0.06 | 0.98 | 0.21 | 0.54 | 0.19 |
| BC163 | 0.19 | 0.09 | 0.05 | 0.06 | 2.08 | 0.56 | 0.03 | 0.05 | 0.04 | 0.24 | 0.2 | 0.33 | 0.14 |
| BC173 | 1.69 | 0.57 | 0.15 | 0.26 | 1.22 | 0.33 | 0.42 | 0.29 | 0.4 | 2.16 | 0.07 | 1.32 | 0.54 |
| BC178 | 0.1 | 0.03 | 0.13 | 0.04 | 2.08 | 0.99 | 0.02 | 0.03 | 0.11 | 0.85 | 0.29 | 1.24 | 0.32 |
| BC186 | 0.22 | 0.07 | 0.11 | 0.07 | 3.02 | 0.37 | 0.05 | 0.05 | 0.04 | 3.77 | 0.2 | 1.16 | 0.53 |
| BC192 | 0.21 | 0.06 | 0.3 | 0.15 | 4.52 | 1.81 | 0.04 | 0.03 | 0.16 | 1.1 | 0.55 | 1.23 | 1.11 |
| BC197 | 0.08 | 0.03 | 0.1 | 0.05 | 0.89 | 0.44 | 0.02 | 0.01 | 0.01 | 0.13 | 0.15 | 0.43 | 0.1 |
| BC199 | 0.99 | 0.41 | 0.07 | 0.11 | 1.16 | 0.11 | 0.13 | 0.11 | 0.16 | 1.28 | 0.2 | 0.73 | 0.33 |
| BC205 | 1.04 | 0.3 | 0.16 | 0.24 | 0.95 | 0.26 | 0.24 | 0.18 | 0.19 | 0.23 | 0.06 | 0.47 | 0.3 |
| BC207 | 1.68 | 0.57 | 0.14 | 0.14 | 1.04 | 0.15 | 0.33 | 0.31 | 0.07 | 0.21 | 0.09 | 0.82 | 0.23 |
| BC216 | 1.19 | 0.46 | 0.29 | 0.43 | 1.07 | 0.15 | 0.28 | 0.16 | 0.36 | 0.19 | 0.08 | 1.46 | 0.34 |
| BC231 | 1.08 | 0.38 | 0.19 | 0.4 | 1.38 | 0.45 | 0.27 | 0.27 | 0.37 | 0.13 | 0.05 | 1.29 | 0.8 |
| BC234 | 1.4 | 0.46 | 0.09 | 0.37 | 1.42 | 0.48 | 0.21 | 0.18 | 0.32 | 0.3 | 0.05 | 1.21 | 0.52 |
| BC242 | 0.79 | 0.31 | 0.16 | 0.24 | 1.41 | 0.38 | 0.2 | 0.12 | 0.35 | 0.14 | 0.05 | 1.05 | 0.33 |
| BC244 | 1.42 | 0.63 | 0.26 | 0.33 | 1.05 | 0.48 | 0.32 | 0.22 | 0.65 | 0.19 | 0.12 | 1.27 | 0.31 |
| BC246 | 0.84 | 0.29 | 0.19 | 0.26 | 0.9 | 0.39 | 0.19 | 0.14 | 0.25 | 0.25 | 0.13 | 0.55 | 0.26 |
| BC253 | 0.84 | 0.5 | 0.08 | 0.25 | 0.95 | 0.32 | 0.21 | 0.27 | 0.65 | 1.34 | 0.04 | 1.02 | 0.25 |
| BC257 | 0.18 | 0.21 | 0.11 | 0.29 | 1.65 | 0.8 | 0.05 | 0.05 | 0 | 1.39 | 0.07 | 0.53 | 0.51 |
| BC235 | 0.66 | 0.62 | 0 | 0.3 | 1.02 | 0.59 | 0.14 | 0.1 | 0.37 | 0.16 | 0.1 | 0.32 | 0.15 |
| BC265 | 0.4 | 0.11 | 0.13 | 0.17 | 0.6 | 0.24 | 0.07 | 0.04 | 0.12 | 0.21 | 0.12 | 0.25 | 0.16 |
| BC270 | 0.18 | 0.06 | 0.14 | 0.08 | 0.76 | 0.34 | 0.03 | 0.03 | 0.06 | 0.59 | 0.1 | 0.7 | 0.16 |
| BC272 | 1.67 | 0.46 | 0.22 | 0.3 | 0.81 | 0.37 | 0.33 | 0.19 | 0.51 | 0.23 | 0.09 | 0.73 | 0.23 |
| BC278 | 0.78 | 0.27 | 0.21 | 0.23 | 1.72 | 0.33 | 0.12 | 0.09 | 0.15 | 0.58 | 0.13 | 1 | 0.48 |
| BC280 | 0.38 | 0.12 | 0.13 | 0.13 | 0.45 | 0.25 | 0.06 | 0.04 | 0.12 | 0.29 | 0.07 | 0.51 | 0.11 |
| BC287 | 0.26 | 0.08 | 0.15 | 0.15 | 0.93 | 0.36 | 0.04 | 0.03 | 0.1 | 0.7 | 0.14 | 0.64 | 0.33 |
| BC211 | 0.18 | 0.09 | 0.13 | 0.17 | 2.64 | 0.95 | 0.02 | 0.02 | 0.12 | 0.42 | 0.16 | 0.23 | 0.22 |
| BC220 | 0.84 | 0.23 | 0.19 | 0.42 | 0.36 | 0.49 | 0.12 | 0.07 | 0.44 | 0.15 | 0.19 | 0.16 | 0.09 |
| BC225 | 0.25 | 0.08 | 0.1 | 0.17 | 0.8 | 0.32 | 0.07 | 0.02 | 0.15 | 0.14 | 0.15 | 0.13 | 0.05 |
| BC233 | 0.89 | 0.45 | 0.08 | 0.42 | 1.02 | 0.34 | 0.22 | 0.17 | 0.35 | 0 | 0.12 | 0.36 | 0.37 |
| BC240 | 0.74 | 0.45 | 0.14 | 0.32 | 0.93 | 0.32 | 0.09 | 0.12 | 0.27 | 1.71 | 0.26 | 0.81 | 0.32 |
| BC266 | 0.25 | 0.08 | 0.12 | 0.08 | 0.85 | 0.74 | 0.04 | 0.04 | 0.18 | 0.26 | 0.23 | 0.31 | 0.17 |
| BC269 | 0.53 | 0.16 | 0.26 | 0.16 | 0.77 | 0.23 | 0.09 | 0.07 | 0.16 | 0.9 | 0.09 | 0.61 | 0.23 |
| BC279 | 0.44 | 0.12 | 0.17 | 0.18 | 1.06 | 0.21 | 0.07 | 0.05 | 0.15 | 0.8 | 0.07 | 0.37 | 0.28 |

## Figure 2

| Pat | let7a | let7e | let7i | miR17 | miR125b | miR222 | let7d | let7f | miR107 | miR194 | miR423 | miR424 | miR660 |
|---|---|---|---|---|---|---|---|---|---|---|---|---|---|
| K39 | 1.91 | 1.01 | 0.39 | 0.36 | 1.16 | 0.15 | 0.75 | 0.75 | 0.5 | 0.66 | 3.34 | 0.36 | 1.79 |
| K40 | 0.58 | 0.25 | 0.24 | 0.12 | 1.07 | 0.26 | 0.19 | 0.19 | 0.1 | 0.15 | 0.37 | 0.19 | 0.51 |
| K41 | 0.5 | 0.2 | 0.26 | 0.13 | 0.81 | 0.29 | 0.11 | 0.11 | 0.09 | 0.11 | 1.62 | 0.15 | 0.53 |
| K42 | 1.57 | 0.67 | 0.25 | 0.3 | 0.76 | 0.08 | 0.56 | 0.56 | 0.31 | 0.4 | 1.94 | 0.13 | 0.88 |
| K43 | 1.39 | 0.52 | 0.37 | 0.26 | 0.88 | 0.21 | 0.46 | 0.46 | 0.21 | 0.41 | 2 | 0.13 | 0.76 |
| K44 | 0.32 | 0.15 | 0.13 | 0.15 | 0.64 | 0.13 | 0.12 | 0.12 | 0.09 | 0.09 | 0.13 | 0.08 | 0.93 |
| K45 | 0.89 | 0.3 | 0.45 | 0.41 | 1.93 | 0.51 | 0.16 | 0.16 | 0.15 | 0.28 | 0.63 | 0.39 | 1.39 |
| K46 | 0.31 | 0.12 | 0.22 | 0.52 | 0.69 | 0.24 | 0.08 | 0.08 | 0.07 | 0.04 | 0.32 | 0.26 | 1.04 |
| K47 | 0.68 | 0.27 | 0.57 | 0.22 | 1.55 | 0.5 | 0.18 | 0.18 | 0.11 | 0.14 | 1.56 | 0.49 | 0.9 |
| K48 | 0.95 | 0.31 | 0.6 | 0.18 | 1.36 | 0.39 | 0.39 | 0.39 | 0.27 | 0.19 | 1.87 | 0.31 | 1.2 |
| K50 | 0.93 | 0.45 | 0.33 | 0.27 | 0.77 | 0.19 | 0.65 | 0.65 | 0.44 | 0.85 | 4.44 | 1.25 | 1.29 |
| K51 | 1.24 | 0.62 | 0.2 | 0.26 | 0.71 | 0.19 | 0.43 | 0.43 | 0.23 | 0.44 | 0.51 | 0.19 | 1.3 |
| K52 | 0.78 | 0.38 | 0.31 | 0.18 | 0.6 | 0.29 | 0.21 | 0.21 | 0.12 | 0.2 | 0.39 | 0.06 | 0.67 |
| K53 | 0.49 | 0.18 | 0.39 | 0.23 | 1.12 | 0.4 | 0.2 | 0.2 | 0.13 | 0.26 | 0.58 | 0.1 | 0.64 |
| K54 | 0.73 | 0.37 | 0.27 | 0.21 | 0.81 | 0.19 | 0.37 | 0.37 | 0.31 | 0.74 | 0.33 | 0.95 | 4.35 |
| K55 | 1.13 | 0.39 | 0.19 | 0.17 | 0.89 | 0.23 | 0.04 | 0.04 | 0.04 | 0.06 | 0.17 | 0.06 | 0.12 |
| K56 | 0.11 | 0.04 | 0.09 | 0.08 | 0.07 | 0.07 | 0.04 | 0.04 | 0.03 | 0.04 | 0.08 | 0.02 | 0.05 |
| K57 | 1.39 | 0.64 | 0.35 | 0.27 | 0.81 | 0.13 | 0.98 | 0.98 | 0.69 | 2.69 | 1.71 | 1.19 | 14.52 |
| K58 | 0.5 | 0.24 | 0.35 | 0.16 | 0.79 | 0.23 | 0.72 | 0.72 | 0.46 | 0.69 | 2.03 | 0.2 | 1.01 |
| K59 | 1.17 | 0.4 | 0.34 | 0.21 | 1.21 | 0.26 | 0.17 | 0.17 | 0.13 | 0.19 | 1.06 | 0.18 | 1.37 |
| K60 | 1.22 | 0.67 | 0.35 | 0.28 | 0.95 | 0.22 | 0.87 | 0.87 | 0.77 | 0.9 | 5.22 | 0.59 | 3.93 |
| K61 | 0.54 | 0.18 | 0.2 | 0.28 | 0.76 | 0.45 | 1.14 | 1.14 | 0.79 | 1.32 | 3.02 | 0.49 | 2.88 |
| K75 | 0.5 | 0.21 | 0.19 | 0.19 | 0.68 | 0.64 | 0 | 0 | 0 | 0.01 | 0 | 0.01 | 0.01 |
| K77 | 3.47 | 1.37 | 0.39 | 0.56 | 1.14 | 0.27 | 0.59 | 0.59 | 0.5 | 0.58 | 1.71 | 0.15 | 2.14 |
| K78 | 2.45 | 0.84 | 0.27 | 0.38 | 0.69 | 0.29 | 1.28 | 1.28 | 0.95 | 0.9 | 2.44 | 0.21 | 3.35 |
| K79 | 3.25 | 1.22 | 0.15 | 0.02 | 0.9 | 0.03 | 0.32 | 0.32 | 0.21 | 0.27 | 0.11 | 0.05 | 0.32 |
| K80 | 0.99 | 0.4 | 0.57 | 0.22 | 1 | 0.67 | 0.16 | 0.16 | 0.13 | 0 | 0.13 | 0.04 | 0.15 |
| K82 | 4.3 | 1.95 | 0.49 | 0.88 | 1.27 | 0.35 | 0.45 | 0.45 | 0.35 | 0.39 | 1.76 | 0.52 | 1.91 |
| K83 | 1.79 | 0.64 | 0.43 | 0.31 | 0.69 | 0.29 | 1.51 | 1.51 | 1.06 | 0.66 | 1.92 | 0.31 | 9.38 |
| K63 | 0.88 | 0.3 | 0.22 | 0.23 | 0.54 | 0.16 | 0.36 | 0.36 | 0.24 | 0.37 | 1.03 | 0.13 | 0.37 |
| K64 | 0.82 | 0.21 | 0.21 | 0.33 | 1.46 | 0.26 | 0.24 | 0.24 | 0.15 | 0.33 | 0.4 | 0.2 | 0.61 |
| K65 | 1.14 | 0.35 | 0.21 | 0.04 | 0.39 | 0.02 | 0.34 | 0.34 | 0.4 | 0.01 | 0.05 | 0.1 | 0.46 |
| K66 | 0.54 | 0.2 | 0.37 | 0.21 | 1.11 | 0.9 | 0.18 | 0.18 | 0.1 | 0.14 | 0.36 | 0.54 | 0.31 |
| K67 | 2.21 | 0.86 | 0.31 | 0.36 | 1.04 | 0.24 | 0.84 | 0.84 | 0.48 | 0.44 | 0.55 | 0.17 | 2.49 |
| K68 | 0.92 | 0.3 | 0.15 | 0.11 | 0.55 | 0.12 | 0.29 | 0.29 | 0.19 | 0.07 | 1.03 | 0.09 | 0.63 |
| K69 | 1.19 | 0.36 | 0.21 | 0.33 | 0.98 | 0.25 | 0.41 | 0.41 | 0.26 | 0.36 | 1.08 | 0.23 | 0.87 |
| K71 | 0.65 | 0.18 | 0.23 | 0.14 | 0.94 | 0.32 | 0.17 | 0.17 | 0.1 | 0.16 | 0.66 | 0.2 | 0.42 |
| K70 | 1.81 | 0.74 | 0.41 | 0.33 | 1.04 | 0.3 | 0.66 | 0.66 | 0.38 | 0.66 | 0.54 | 0.23 | 1.52 |
| K72 | 1.75 | 0.56 | 0.26 | 0.38 | 0.86 | 0.16 | 0.74 | 0.74 | 0.46 | 0.65 | 0.66 | 0.21 | 1.29 |
| K73 | 0.7 | 0.19 | 0.3 | 0.23 | 1.6 | 0.57 | 0.16 | 0.16 | 0.12 | 0.12 | 0.35 | 0.24 | 0.55 |

**Figure 3**

## Figure 4

```
  ┌────────┐   ┌────────┐ ┌────────────────┐ ┌────────┐
  │ Urine  │   │ Serum  │ │ amniotic liquor│ │ Ascites│
  └───┬────┘   └───┬────┘ └───────┬────────┘ └───┬────┘
      │            └──────────────┼──────────────┘
      │                    ┌──────┴───────┐
      │                    │dilute with DPBS│
      │                    └──────┬───────┘
      │                           │
      └───────────┬───────────────┘
          ┌───────┴────────┐
          │ extrude through│
          │ syringe filter │
          └───────┬────────┘
          ┌───────┴────────┐
          │ wash filter with│
          │     DPBS        │
          └───────┬────────┘
          ┌───────┴────────┐
          │  elution with  │
          │  appropriate   │
          │   lysisbuffer  │
          └────────────────┘
```

## Figure 5

● 0.20 µm CA filter

● 0.22µm Nylon filter

**Figure 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 3070178 A1
- US 4883750 A
- US 5912148 A
- US 5843650 A
- US 5846709 A
- US 5846783 A
- US 5849546 A
- US 5849497 A
- US 5849547 A
- US 5858652 A
- US 5866366 A
- US 5916776 A
- US 5922574 A
- US 5928905 A
- US 5928906 A
- US 5932451 A
- US 5935825 A
- US 5939291 A
- US 5942391 A
- GB 2202328 A
- US 8901025 W

### Non-patent literature cited in the description

- **LANCET.** Global, regional, and national incidence, prevalence, and years lived with disability for 328 diseases and injuries for 195 countries, 1990-2016: a systematic analysis for the Global Burden of Disease Study 2016. *Disease, G.B.D., I. Injury, and C. Prevalence,* 2017, vol. 390 (10100), 1211-1259
- **SHARMA, R.** Breast cancer incidence, mortality and mortality-to-incidence ratio (MIR) are associated with human development, 1990-2016: evidence from Global Burden of Disease Study 2016. *Breast Cancer,* 2019
- Epidemiological characteristics of and risk factors for breast cancer in the world. **MOMENIMOVAHED, Z. ; H. SALEHINIYA.** Breast Cancer. Dove Med Press, 2019, vol. 11, 151-164
- **OEFFINGER, K.C. et al.** Breast Cancer Screening for Women at Average Risk: 2015 Guideline Update From the American Cancer Society. *JAMA,* 2015, vol. 314 (15), 1599-614
- **TABAR, L. et al.** Insights from the breast cancer screening trials: how screening affects the natural history of breast cancer and implications for evaluating service screening programs. *Breast J,* 2015, vol. 21 (1), 13-20
- **WOCKEL, A. et al.** Interdisciplinary Screening, Diagnosis, Therapy and Follow-up of Breast Cancer. Guideline of the DGGG and the DKG (S3-Level, AWMF Registry Number 032/045OL, December 2017) - Part 1 with Recommendations for the Screening, Diagnosis and Therapy of Breast Cancer. *Geburtshilfe Frauenheilkd,* 2018, vol. 78 (10), 927-948
- **SANKARANARAYANAN, R. et al.** Cancer survival in Africa, Asia, and Central America: a population-based study. *Lancet Oncol,* 2010, vol. 11 (2), 165-73
- **KOLB, T.M. ; J. LICHY ; J.H. NEWHOUSE.** Comparison of the performance of screening mammography, physical examination, and breast US and evaluation of factors that influence them: an analysis of 27,825 patient evaluations. *Radiology,* 2002, vol. 225 (1), 165-75
- **WANG, L.** Early Diagnosis of Breast Cancer. *Sensors (Basel),* 2017, vol. 17 (7
- **ARNETH, B.** Update on the types and usage of liquid biopsies in the clinical setting: a systematic review. *BMC Cancer,* 2018, vol. 18 (1), 527
- **SALEHI, M. ; M. SHARIFI.** Exosomal miRNAs as novel cancer biomarkers: Challenges and opportunities. *J Cell Physiol,* 2018, vol. 233 (9), 6370-6380
- **ERBES, T. et al.** Feasibility of urinary microRNA detection in breast cancer patients and its potential as an innovative non-invasive biomarker. *BMC Cancer,* 2015, vol. 15, 193
- **BALACESCU, O. et al.** Urinary microRNAs for prostate cancer diagnosis, prognosis, and treatment response: are we there yet?. *Wiley Interdiscip Rev RNA,* 2017, vol. 8 (6
- **GASPARRI, M.L. et al.** Beyond circulating microRNA biomarkers: Urinary microRNAs in ovarian and breast cancer. *Tumour Biol,* 2017, vol. 39 (5), 1010428317695525

- **GRAYSON, K. et al.** Urine Biomarkers for the Early Detection of Ovarian Cancer - Are We There Yet?. *Biomark Cancer,* 2019, vol. 11, 1179299X19830977
- **FRANZEN, C.A. et al.** Urinary Exosomes: The Potential for Biomarker Utility, Intercellular Signaling and Therapeutics in Urological Malignancy. *J Urol,* 2016, vol. 195 (5), 1331-1339
- **BALZEAU, J. et al.** The LIN28/let-7 Pathway in Cancer. *Front Genet,* 2017, vol. 8, 31
- **DUFRESNE, S. et al.** A Review of Physical Activity and Circulating miRNA Expression: Implications in Cancer Risk and Progression. *Cancer Epidemiol Biomarkers Prev,* 2018, vol. 27 (1), 11-24
- **ELGHOROURY, E.A. et al.** Evaluation of miRNA-21 and miRNA Let-7 as Prognostic Markers in Patients With Breast Cancer. *Clin Breast Cancer,* 2018, vol. 18 (4), e721-e726
- **MASOOD, N. et al.** Entangling Relation of Micro RNA-let7, miRNA-200 and miRNA-125 with Various Cancers. *Pathol Oncol Res,* 2017, vol. 23 (4), 707-715
- **SEN, C.K. et al.** Micromanaging vascular biology: tiny microRNAs play big band. *J Vasc Res,* 2009, vol. 46 (6), 527-40
- **THAMMAIAH, C.K. ; S. JAYARAM.** Role of let-7 family microRNA in breast cancer. *Noncoding RNA Res,* 2016, vol. 1 (1), 77-82
- **DE ANDA-JAUREGUI, G. et al.** Nonredundant, Highly Connected MicroRNAs Control Functionality in Breast Cancer Networks. *Int J Genomics,* 2018, vol. 2018, 9585383
- **LEHMANN, T.P. et al.** Relative levels oflet-7a, miR-17, miR-27b, miR-125a, miR-125b and miR-206 as potential molecular markers to evaluate grade, receptor status and molecular type in breast cancer. *Mol Med Rep,* 2015, vol. 12 (3), 4692-4702
- **SUN, H. et al.** Let7 miRNAs sensitize breast cancer stem cells to radiationinduced repression through inhibition of the cyclin D1/Akt1/Wnt1 signaling pathway. *Mol Med Rep,* 2016, vol. 14 (4), 3285-92
- **BOBBILI, M.R. et al.** OncomiR-17-5p: alarm signal in cancer?. *Oncotarget,* 2017, vol. 8 (41), 71206-71222
- **HESARI, A. et al.** Expression of circulating miR-17, miR-25, and miR-133 in breast cancer patients. *J Cell Biochem,* 2018
- **LI, J. et al.** miR-17-5p suppresses cell proliferation and invasion by targeting ETV1 in triple- negative breast cancer. *BMC Cancer,* 2017, vol. 17 (1), 745
- **SWELLAM, M. et al.** Role of some circulating MiRNAs on breast cancer diagnosis. *Arch Physiol Biochem,* 2018, 1-9
- **WANG, Y. et al.** MiR-17-5p may serve as a novel predictor for breast cancer recurrence. *Cancer Biomark,* 2018, vol. 22 (4), 721-726
- **FELICIANO, A. et al.** miR-125b acts as a tumor suppressor in breast tumorigenesis via its novel direct targets ENPEP, CK2-alpha, CCNJ, and MEGF9. *PLoS One,* 2013, vol. 8 (10), e76247
- **FERRACIN, M. et al.** miR-125b targets erythropoietin and its receptor and their expression correlates with metastatic potential and ERBB2/HER2 expression. *Mol Cancer,* 2013, vol. 12 (1), 130
- **LUO, Y. et al.** Elevated microRNA-125b levels predict a worse prognosis in HER2-positive breast cancer patients. *Oncol Lett,* 2017, vol. 13 (2), 867-874
- **MAR-AGUILAR, F. et al.** Differential expression of miR-21, miR-125b and miR-191 in breast cancer tissue. *Asia Pac J Clin Oncol,* 2013, vol. 9 (1), 53-9
- **AMINI, S. et al.** Expression Analysis of MicroRNA-222 in Breast Cancer. *Clin Lab,* 2018, vol. 64 (4), 491-496
- **LIU, S. et al.** miR-221/222 activate the Wnt/beta-catenin signaling to promote triple-negative breast cancer. *J Mol Cell Biol,* 2018, vol. 10 (4), 302-315
- **LIANG, Y.K. et al.** MiR-221/222 promote epithelial-mesenchymal transition by targeting Notch3 in breast cancer cell lines. *NPJ Breast Cancer,* 2018, vol. 4, 20
- **HAN, S.H. et al.** MicroRNA-222 Expression as a Predictive Marker for Tumor Progression in Hormone Receptor-Positive Breast Cancer. *J Breast Cancer,* 2017, vol. 20 (1), 35-44
- **DING, J. et al.** Exosome-mediated miR-222 transferring: An insight into NF-kappaB-mediated breast cancer metastasis. *Exp Cell Res,* 2018, vol. 369 (1), 129-138
- **GAO, B. et al.** MicroRNA-107 is downregulated and having tumor suppressive effect in breast cancer by negatively regulating brain-derived neurotrophic factor. *J Gene Med,* 2017, vol. 19 (12
- **PETROVIC, N. et al.** MicroRNA in breast cancer: The association with BRCA1/2. *Cancer Biomark,* 2017, vol. 19 (2), 119-128
- **AI, H. et al.** microRNAs-107 inhibited autophagy, proliferation, and migration of breast cancer cells by targeting HMGB1. *J Cell Biochem,* 2018
- **ZHANG, L. et al.** MiR-107 down-regulates SIAH1 expression in human breast cancer cells and silencing of miR-107 inhibits tumor growth in a nude mouse model of triple-negative breast cancer. *Mol Carcinog,* 2016, vol. 55 (5), 768-77
- **LI, X.Y. et al.** MiRNA-107 inhibits proliferation and migration by targeting CDK8 in breast cancer. *Int J Clin Exp Med,* 2014, vol. 7 (1), 32-40
- **KLEIVI SAHLBERG, K. et al.** A serum microRNA signature predicts tumor relapse and survival in triple-negative breast cancer patients. *Clin Cancer Res,* 2015, vol. 21 (5), 1207-14

- **STUCKRATH, I. et al.** Aberrant plasma levels of circulating miR-16, miR-107, miR-130a and miR-146a are associated with lymph node metastasis and receptor status of breast cancer patients. *Oncotarget,* 2015, vol. 6 (15), 13387-401
- **YANG, F. ; Z. XIAO ; S. ZHANG.** Knockdown of miR-194-5p inhibits cell proliferation, migration and invasion in breast cancer by regulating the Wnt/beta-catenin signaling pathway. *Int J Mol Med,* 2018, vol. 42 (6), 3355-3363
- **HUO, D. et al.** Identification of a circulating microRNA signature to distinguish recurrence in breast cancer patients. *Oncotarget,* 2016, vol. 7 (34), 55231-55248
- **LE, X.F. et al.** Modulation of MicroRNA-194 and cell migration by HER2-targeting trastuzumab in breast cancer. *PLoS One,* 2012, vol. 7 (7), e41170
- **ZHAO, H et al.** Genetic analysis and preliminary function study of miR-423 in breast cancer. *Tumour Biol,* 2015, vol. 36 (6), 4763-71
- **SMITH, R.A et al.** A genetic variant located in miR-423 is associated with reduced breast cancer risk. *Cancer Genomics Proteomics,* 2012, vol. 9 (3), 115-8
- **MURRIA ESTAL, R. et al.** MicroRNA signatures in hereditary breast cancer. *Breast Cancer Res Treat,* 2013, vol. 142 (1), 19-30
- **WANG, J. et al.** miR-424-5p regulates cell proliferation, migration and invasion by targeting doublecortin-like kinase 1 in basal-like breast cancer. *Biomed Pharmacother,* 2018, vol. 102, 147-152
- **RODRIGUEZ-BARRUECO, R. et al.** miR-424(322)/503 is a breast cancer tumor suppressor whose loss promotes resistance to chemotherapy. *Genes Dev,* 2017, vol. 31 (6), 553-566
- **ZHANG, L. et al.** A circulating miRNA signature as a diagnostic biomarker for non-invasive early detection of breast cancer. *Breast Cancer Res Treat,* 2015, vol. 154 (2), 423-34
- **SHEN, Y. et al.** Inhibition of miR-660-5p expression suppresses tumor development and metastasis in human breast cancer. *Genet Mol Res,* 2017, vol. 16 (1
- **KRISHNAN, P. et al.** Next generation sequencing profiling identifies miR-574-3p and miR-660-5p as potential novel prognostic markers for breast cancer. *BMC Genomics,* 2015, vol. 16, 735
- Total RNA Purification Kit Product Insert Product # 17200, 37500, 17250. *Norgen-Biotek-Corp,* 2018, https://norqenbiotek.com/sites/default/files/resources/Total%20RNA%20Purification%20Kit%20Insert%20PI17200-33.pdf.
- **BUSK, P.K.** A tool for design of primers for microRNA-specific quantitative RT-qPCR. *BMC Bioinformatics,* 2014, vol. 15, 29
- **MARABITA, F. et al.** Normalization of circulating microRNA expression data obtained by quantitative real-time RT-PCR. *Brief Bioinform,* 2016, vol. 17 (2), 204-12
- **TUTZ, G. ; H. BINDER.** Generalized additive modeling with implicit variable selection by likelihood-based boosting. *Biometrics,* 2006, vol. 62 (4), 961-71
- R: A language and environment for statistical computing. *R-Core-Team,* 2018, https.-//www.r-project.org/.
- **GHOSH, R.K. ; T. PANDEY ; P. DEY.** Liquid biopsy: A new avenue in pathology. *Cytopathology,* 2019, vol. 30 (2), 138-143
- **MADER, S. ; K. PANTEL.** Liquid Biopsy: Current Status and Future Perspectives. *Oncol Res Treat,* 2017, vol. 40 (7-8), 404-408
- **PANTEL, K. ; C. ALIX-PANABIERES.** Liquid biopsy and minimal residual disease - latest advances and implications for cure. *Nat Rev Clin Oncol,* 2019, vol. 16 (7), 409-424
- **ROSSI, G. ; M. IGNATIADIS.** Promises and Pitfalls of Using Liquid Biopsy for Precision Medicine. *Cancer Res,* 2019, vol. 79 (11), 2798-2804
- **ALIMIRZAIE, S. ; M. BAGHERZADEH ; M.R. AKBARI.** Liquid biopsy in breast cancer: A comprehensive review. *Clin Genet,* 2019, vol. 95 (6), 643-660
- **WANG, H.X. ; G. OLIVIER.** Tumor-derived extracellular vesicles in breast cancer: from bench to bedside. *Cancer Lett,* 2019
- **ZUBOR, P. et al.** Why the Gold Standard Approach by Mammography Demands Extension by Multiomics? Application of Liquid Biopsy miRNA Profiles to Breast Cancer Disease Management. *Int J Mol Sci,* 2019, vol. 20 (12
- **BERETOV, J. et al.** Proteomic Analysis of Urine to Identify Breast Cancer Biomarker Candidates Using a Label-Free LC-MS/MS Approach. *PLoS One,* 2015, vol. 10 (11), e0141876
- **BURTON, C. et al.** Urinary metallomics as a novel biomarker discovery platform: Breast cancer as a case study. *Clin Chim Acta,* 2016, vol. 452, 142-8
- **GUO, C. et al.** Discriminating patients with early-stage breast cancer from benign lesions by detection of oxidative DNA damage biomarker in urine. *Oncotarget,* 2017, vol. 8 (32), 53100-53109
- **HERMAN-SAFFAR, O. et al.** Early non-invasive detection of breast cancer using exhaled breath and urine analysis. *Comput Biol Med,* 2018, vol. 96, 227-232
- **WOOLLAM, M. et al.** Detection of Volatile Organic Compounds (VOCs) in Urine via Gas Chromatography-Mass Spectrometry QTOF to Differentiate Between Localized and Metastatic Models of Breast Cancer. *Sci Rep,* 2019, vol. 9 (1), 2526

- **HOFBAUER, S. L. et al.** A urinary microRNA (miR) signature for diagnosis of bladder cancer. *Urol Oncol,* 2018, vol. 36 (12), 531 e1-531 e8
- **KUTWIN, P. et al.** Urine miRNA as a potential biomarker for bladder cancer detection - a meta-analysis. *Cent European J Urol,* 2018, vol. 71 (2), 177-185
- **LEKCHNOV, E.A. et al.** Searching for the Novel Specific Predictors of Prostate Cancer in Urine: The Analysis of 84 miRNA Expression. *Int J Mol Sci,* 2018, vol. 19 (12
- **MOLDOVAN, L. et al.** Methodological challenges in utilizing miRNAs as circulating biomarkers. *J Cell Mol Med,* 2014, vol. 18 (3), 371-90
- **LIU, J. et al.** Tumour-suppressive microRNA-424-5p directly targets CCNE1 as potential prognostic markers in epithelial ovarian cancer. *Cell Cycle,* 2018, vol. 17 (3), 309-318
- **WEI, S. et al.** miR-424-5p promotes proliferation of gastric cancer by targeting Smad3 through TGF-beta signaling pathway. *Oncotarget,* 2016, vol. 7 (46), 75185-75196
- **WU, K. et al.** MicroRNA-424-5p suppresses the expression of SOCS6 in pancreatic cancer. *Pathol Oncol Res,* 2013, vol. 19 (4), 739-48
- **XU, J. et al.** Suppressed miR-424 expression via upregulation of target gene Chk1 contributes to the progression of cervical cancer. *Oncogene,* 2013, vol. 32 (8), 976-87
- **XIE, D. et al.** MicroRNA424 serves an antioncogenic role by targeting cyclindependent kinase 1 in breast cancer cells. *Oncol Rep,* 2018, vol. 40 (6), 3416-3426
- **SHIVAPURKAR, N. et al.** Circulating microRNAs in patients with hormone receptor-positive, metastatic breast cancer treated with dovitinib. *Clin Transl Med,* 2017, vol. 6 (1), 37
- **FORTUNATO, O et al.** Mir-660 is downregulated in lung cancer patients and its replacement inhibits lung tumorigenesis by targeting MDM2-p53 interaction. *Cell Death Dis,* 2014, vol. 5, e1564
- **HE, T. et al.** miR6605p is associated with cell migration, invasion, proliferation and apoptosis in renal cell carcinoma. *Mol Med Rep,* 2018, vol. 17 (1), 2051-2060
- **HE, X. ; Y. SHU.** RNA N6-methyladenosine modification participates in miR-660/E2F3 axis- mediated inhibition of cell proliferation in gastric cancer. *Pathol Res Pract,* 2019, vol. 215 (6), 152393
- **BORZI, C. et al.** mir-660-p53-mir-486 Network: A New Key Regulatory Pathway in Lung Tumorigenesis. *Int J Mol Sci,* 2017, vol. 18 (1
- **ZHANG, P. et al.** Downregulation of microRNA660 inhibits cell proliferation and invasion in osteosarcoma by directly targeting forkhead box O1. *Mol Med Rep,* 2018, vol. 18 (2), 2433-2440
- **ZHOU, C. et al.** Combination of serum miRNAs with Cyfra21-1 for the diagnosis of non-small cell lung cancer. *Cancer Lett,* 2015, vol. 367 (2), 138-46
- **ELKHADRAGY, L. et al.** A regulatory BMI1/let-7i/ERK3 pathway controls the motility of head and neck cancer cells. *Mol Oncol,* 2017, vol. 11 (2), 194-207
- **GADDUCCI, A. et al.** Micro-RNAs and ovarian cancer: the state of art and perspectives of clinical research. *Gynecol Endocrinol,* 2014, vol. 30 (4), 266-71
- **HUR, K. et al.** Identification of a metastasis-specific MicroRNA signature in human colorectal cancer. *J Natl Cancer Inst,* 2015, vol. 107 (3
- **QIN, M.M. et al.** let-7i inhibits proliferation and migration of bladder cancer cells by targeting HMGA1. *BMC Urol,* 2019, vol. 19 (1), 53
- **XIE, J. et al.** miR-7 inhibits the invasion and metastasis of gastric cancer cells by suppressing epidermal growth factor receptor expression. *Oncol Rep,* 2014, vol. 31 (4), 1715-22
- **YANG, W.H. et al.** Repression of bone morphogenetic protein 4 by let-7i attenuates mesenchymal migration of head and neck cancer cells. *Biochem Biophys Res Commun,* 2013, vol. 433 (1), 24-30
- **MAHN, R. et al.** Circulating microRNAs (miRNA) in serum of patients with prostate cancer. *Urology,* 2011, vol. 77 (5), 1265 e9-16
- **XIAO, D. et al.** Melanoma cell-derived exosomes promote epithelial-mesenchymal transition in primary melanocytes through paracrine/autocrine signaling in the tumor microenvironment. *Cancer Lett,* 2016, vol. 376 (2), 318-27
- **CHAKRABORTY, C. ; S. DAS.** Profiling cell-free and circulating miRNA: a clinical diagnostic tool for different cancers. *Tumour Biol,* 2016, vol. 37 (5), 5705-14
- **ZHU, Y. et al.** Identification of a serum microRNA expression signature for detection of lung cancer, involving miR-23b, miR-221, miR-148b and miR-423-3p. *Lung Cancer,* 2017, vol. 114, 6-11
- **DU, W. ; Z. FENG ; Q. SUN.** LncRNA LINC00319 accelerates ovarian cancer progression through miR-423-5p/NACC1 pathway. *Biochem Biophys Res Commun,* 2018, vol. 507 (1-4), 198-202
- **LIN, H. et al.** Inhibition of miR-423-5p suppressed prostate cancer through targeting GRIM-19. *Gene,* 2019, vol. 688, 93-97
- **YANG, H. et al.** Exosomal miR-423-5p targets SUFU to promote cancer growth and metastasis and serves as a novel marker for gastric cancer. *Mol Carcinog,* 2018, vol. 57 (9), 1223-1236
- **MCDERMOTT, A.M. et al.** Identification and validation of oncologic miRNA biomarkers for luminal A-like breast cancer. *PLoS One,* 2014, vol. 9 (1), e87032

- **DING, H.X. et al.** MiRNA Polymorphisms and Cancer Prognosis: A Systematic Review and Meta-Analysis. *Front Oncol,* 2018, vol. 8, 596
- **CAMPOS-PARRA, A.D. et al.** Micro-RNAs as Potential Predictors of Response to Breast Cancer Systemic Therapy: Future Clinical Implications. *Int J Mol Sci,* 2017, vol. 18 (6
- **LIU, B. et al.** Serum miR-21 and miR-125b as markers predicting neoadjuvant chemotherapy response and prognosis in stage II/III breast cancer. *Hum Pathol,* 2017, vol. 64, 44-52
- **XIE, X. et al.** The role of miR-125b-mitochondria-caspase-3 pathway in doxorubicin resistance and therapy in human breast cancer. *Tumour Biol,* 2015, vol. 36 (9), 7185-94
- **HONG, L. et al.** miR-125b inhibited epithelial-mesenchymal transition of triple-negative breast cancer by targeting MAP2K7. *Onco Targets Ther,* 2016, vol. 9, 2639-48
- **MATAMALA, N. et al.** Tumor microRNA expression profiling identifies circulating microRNAs for early breast cancer detection. *Clin Chem,* 2015, vol. 61 (8), 1098-106
- **DAS, R. et al.** MicroRNA-194 Promotes Prostate Cancer Metastasis by Inhibiting SOCS2. *Cancer Res,* 2017, vol. 77 (4), 1021-1034
- **KONG, Q. et al.** MicroRNA-194 suppresses prostate cancer migration and invasion by downregulating human nuclear distribution protein. *Oncol Rep,* 2017, vol. 37 (2), 803-812
- **PENG, Y. et al.** MiRNA-194 activates the Wnt/beta-catenin signaling pathway in gastric cancer by targeting the negative Wnt regulator, SUFU. *Cancer Lett,* 2017, vol. 385, 117-127
- **ZHANG, X. et al.** MicroRNA-194 represses glioma cell epithelialtomesenchymal transition by targeting Bmi1. *Oncol Rep,* 2017, vol. 37 (3), 1593-1600
- **CHEN, Y. et al.** Promotional effect of microRNA-194 on breast cancer cells via targeting F- box/WD repeat-containing protein 7. *Oncol Lett,* 2018, vol. 15 (4), 4439-4444
- **GILLES, M.E. ; F.J. SLACK.** Let-7 microRNA as a potential therapeutic target with implications for immunotherapy. *Expert Opin Ther Targets,* 2018, vol. 22 (11), 929-939
- **CHERNYI, V.S. et al.** Search of MicroRNAs Regulating the Receptor Status of Breast Cancer In Silico and Experimental Confirmation of Their Expression in Tumors. *Bull Exp Biol Med,* 2017, vol. 163 (5), 655-659
- **SUETA, A et al.** Differential expression of exosomal miRNAs between breast cancer patients with and without recurrence. *Oncotarget,* 2017, vol. 8 (41), 69934-69944
- **ZAVESKY, L. et al.** Supernatant versus exosomal urinary microRNAs. Two fractions with different outcomes in gynaecological cancers. *Neoplasma,* 2016, vol. 63 (1), 121-32
- **ARMAND-LABIT, V. ; A. PRADINES.** Circulating cell-free microRNAs as clinical cancer biomarkers. *Biomol Concepts,* 2017, vol. 8 (2), 61-81
- **NEDAEINIA, R. et al.** Circulating exosomes and exosomal microRNAs as biomarkers in gastrointestinal cancer. *Cancer Gene Ther,* 2017, vol. 24 (2), 48-56
- **PRITCHARD, C.C. et al.** Blood cell origin of circulating microRNAs: a cautionary note for cancer biomarker studies. *Cancer Prev Res (Phila),* 2012, vol. 5 (3), 492-497
- **ZHANG, J. et al.** Exosome and exosomal microRNA: trafficking, sorting, and function. *Genomics Proteomics Bioinformatics,* 2015, vol. 13 (1), 17-24
- **LARSSEN, P. et al.** Tracing Cellular Origin of Human Exosomes Using Multiplex Proximity Extension Assays. *Mol Cell Proteomics,* 2017, vol. 16 (3), 502-511
- **KREGER, B.T. et al.** Microvesicle Cargo and Function Changes upon Induction of Cellular Transformation. *J Biol Chem,* 2016, vol. 291 (38), 19774-85
- **TRICARICO, C. ; J. CLANCY ; C. D'SOUZA-SCHOREY.** Biology and biogenesis of shed microvesicles. *Small GTPases,* 2017, vol. 8 (4), 220-232
- **COLOMBO, M. ; G. RAPOSO ; C. THERY.** Biogenesis, secretion, and intercellular interactions of exosomes and other extracellular vesicles. *Annu Rev Cell Dev Biol,* 2014, vol. 30, 255-89
- **BAKHSHANDEH, B. ; M.A. KAMALEDDIN ; K. AALISHAH.** A Comprehensive Review on Exosomes and Microvesicles as Epigenetic Factors. *Curr Stem Cell Res Ther,* 2017, vol. 12 (1), 31-36
- **VITKOVA, V. ; J. ZIVNY ; J. JANOTA.** Endothelial cell-derived microvesicles: potential mediators and biomarkers of pathologic processes. *Biomark Med,* 2018, vol. 12 (2), 161-175
- **POL, E. et al.** Recent developments in the nomenclature, presence, isolation, detection and clinical impact of extracellular vesicles. *J Thromb Haemost,* 2016, vol. 14 (1), 48-56
- **KOWAL, J. et al.** Proteomic comparison defines novel markers to characterize heterogeneous populations of extracellular vesicle subtypes. *Proc Natl Acad Sci U S A,* 2016, vol. 113 (8), E968-77
- **COCUCCI, E. ; J. MELDOLESI.** Ectosomes and exosomes: shedding the confusion between extracellular vesicles. *Trends Cell Biol,* 2015, vol. 25 (6), 364-72
- **MINCIACCHI, V.R. ; M.R. FREEMAN ; D. DI VIZIO.** Extracellular vesicles in cancer: exosomes, microvesicles and the emerging role of large oncosomes. *Semin Cell Dev Biol,* 2015, vol. 40, 41-51
- **GOPAL, S.K. et al.** Extracellular vesicles: their role in cancer biology and epithelial-mesenchymal transition. *Biochem J,* 2017, vol. 474 (1), 21-45

- **XU, R. et al.** Extracellular vesicle isolation and characterization: toward clinical application. *J Clin Invest,* 2016, vol. 126 (4), 1152-62
- **CHEN, Y. et al.** Extracellular vesicles as novel biomarkers and pharmaceutic targets of diseases. *Acta Pharmacol Sin,* 2018, vol. 39 (4), 499-500
- **VALENCIA, K. ; F. LECANDA.** Microvesicles: Isolation, Characterization for In Vitro and In Vivo Procedures. *Methods Mol Biol,* 2016, vol. 1372, 181-92
- **ERDBRUGGER, U. ; J. LANNIGAN.** Analytical challenges of extracellular vesicle detection: A comparison of different techniques. *Cytometry A,* 2016, vol. 89 (2), 123-34
- **CHOI, D.S. et al.** Proteomics of extracellular vesicles: Exosomes and ectosomes. *Mass Spectrom Rev,* 2015, vol. 34 (4), 474-90
- **CHOI, D.S. et al.** Proteomics, transcriptomics and lipidomics of exosomes and ectosomes. *Proteomics,* 2013, vol. 13 (10-11), 1554-71
- **ENDERLE, D et al.** Characterization of RNA from Exosomes and Other Extracellular Vesicles Isolated by a Novel Spin Column-Based Method. *PLoS One,* 2015, vol. 10 (8), e0136133
- **CHIRIACO, M.S. et al.** Lab-on-Chip for Exosomes and Microvesicles Detection and Characterization. *Sensors (Basel),* 2018, vol. 18 (10
- **SZATANEK, R. et al.** Isolation of extracellular vesicles: Determining the correct approach (Review). *Int J Mol Med,* 2015, vol. 36 (1), 11-7
- **YUANA, Y. et al.** Handling and storage of human body fluids for analysis of extracellular vesicles. *J Extracell Vesicles,* 2015, vol. 4, 29260
- **GIEBEL, B. ; C. HELMBRECHT.** Methods to Analyze EVs. *Methods Mol Biol,* 2017, vol. 1545, 1-20
- **KEERTHIKUMAR, S. et al.** Bioinformatics Tools for Extracellular Vesicles Research. *Methods Mol Biol,* 2017, vol. 1545, 189-196
- **YUANA, Y. ; A. STURK ; R. NIEUWLAND.** Extracellular vesicles in physiological and pathological conditions. *Blood Rev,* 2013, vol. 27 (1), 31-9
- **BOING, A.N. et al.** Single-step isolation of extracellular vesicles by size-exclusion chromatography. *J Extracell Vesicles,* 2014, vol. 3
- **COUMANS, F.A. et al.** Reproducible extracellular vesicle size and concentration determination with tunable resistive pulse sensing. *J Extracell Vesicles,* 2014, vol. 3, 25922
- **GRAY, W.D. ; A.J. MITCHELL ; C.D. SEARLES.** An accurate, precise method for general labeling of extracellular vesicles. *MethodsX,* 2015, vol. 2, 360-7
- **MRVAR-BRECKO, A. et al.** Isolated microvesicles from peripheral blood and body fluids as observed by scanning electron microscope. *Blood Cells Mol Dis,* 2010, vol. 44 (4), 307-12
- **SUN, Y. et al.** Facile preparation of salivary extracellular vesicles for cancer proteomics. *Sci Rep,* 2016, vol. 6, 24669
- **YUANA, Y. et al.** Co-isolation of extracellular vesicles and high-density lipoproteins using density gradient ultracentrifugation. *J Extracell Vesicles,* 2014, vol. 3
- I. WMA Declaration of Helsinki - Ethical Principles for Medical Research Involving Human Subjects. *The World Medical Association,* 2018, https://www.wma.net/policies-post/wma-declaration-of-helsinki-ethical-principles-for-medical-research-involving-human-subjects/.
- **HAAN, C. ; I. BEHRMANN.** A cost effective non-commercial ECL-solution for Western blot detections yielding strong signals and low background. *J Immunol Methods,* 2007, vol. 318 (1-2), 11-9